# EUROPEAN PATENT APPLICATION

(11) **EP 4 368 730 A2**
(43) Date of publication of application: **15.05.2024**
(21) Application number: 24152595.5
(22) Date of filing: 05.10.2020
(51) Int. Cl.: C12Q 1/689

(54) **MICROBIOME INTERVENTIONS**

(30) Priority: 04.10.2019 GB 201914385
(62) Divisional of application: 20803681.4
(71) Applicant: MARS INCORPORATED, McLean, VA 22101 (US)
(72) Inventor: MARSHALL-JONES, Zoe, Leicestershire, LE14 4RT (GB); HAYDOCK, Richard, Leicestershire, LE14 4RT (GB); O'FLYNN, Ciaran, Leicestershire, LE14 4RT (GB); MOLINA, Luis, 30470 Aimargues (FR); QUEAU, Yann, 30470 Aimargues (FR); IGARASHI, Hirotaka, Sagamihara, 252-5201 (JP)
(74) Representative: Carpmaels & Ransford LLP

(57) **Abstract**

The present disclosure relates to compositions comprising a range of ingredients suitable for use in adjusting and/or treating a companion animal such as a canid (e.g. a dog) or a feline (e.g. a cat) and their microbiomes, monitoring tools, and diagnostic methods for determining the health of a companion animal and their microbiome.

## Description

### TECHNICAL FIELD

The present disclosure relates to compositions, use of the compositions, and methods for adjusting and/or treating companion animals and their microbiomes, monitoring tools, and diagnostic methods for determining the health of a companion animal and their microbiome.

### BACKGROUND TO THE INVENTION

The microbiome is described as all of the microorganisms in any particular environment and is more specifically the combined genetic material of the microorganisms in that environment. In mammals the microbes exist in a symbiotic relationship with their host being present on the skin, in the gut and in the oral cavity and indeed these microorganisms play an important role in the host's health forming a barrier to colonisation with foreign microbes and hence protecting the animal against pathogens as well as in the gut aiding the breakdown of nutrients releasing energy and producing vitamins essential to life.

Although the influence of pathogenic and probiotic microbes on their mammalian hosts have long been appreciated there is now a growing appreciation of the influence of the total community composition the detailed microbial balance and the genetic potential of the inhabiting microbiotaNon-invasive studies of the gut microbiome in humans are predominantly conducted using a faecal surrogate samples that are particularly representative of the distal colon, the major site of bacterial growth within the intestinal tract. Bacterial growth in the colon occurs through fermentation, a process using dietary ingredients that remain undigested by the host and hence are not absorbed being left available for influencing the gut microbial communities and the microbiome.

Development of the microbiome occurs shortly following birth in mammals though a combination of maternal or parental and environmental inoculation and the interplay or bacteria with the immune system is considered to play a role in immune priming for future recognition of pathogenic microbes and intestinal physiology. Based on studies in human infants these early developments in the gut microbiome are now considered to impact health throughout later life. In the weeks and months following birth a rapid increase in diversity occurs representing the early establishment of gut microbiome development during this stage the microbiome and microbial communities existing are considered to be plastic or malleable. In part as a result of this pre-diversity development stage in the early lifestages, puppies have an under-developed gut barrier, which includes the gastrointestinal microbiome as well as histological and gut associated immune functions. Puppies and young dogs are therefore are more prone to gastrointestinal illnesses such as soft malformed faeces, diarrhoea, flatulence and sickness. The increase in diversity during maturation leads to the development of an adult gut microbiome that can be resilient to colonisation even by beneficial microbes such as probiotic species and hence the adult microbiome is relatively resilient resisting large shifts in community structure and intestinal dysbiosis. The microbiome of adult animals contains similar bacterial communities, but is more diverse and well developed with an adjusted community structure representing a more robust and resilient microbiota with a gut microbiome showing enhanced resilience compared to other lifestages.

Senior and geriatric dogs and people are also more prone to digestive issues such as diarrhoea and gastrointestinal infections which can have heightened severity and greater implications. These gastrointestinal issues may occur in part as a result of a deterioration in the gut microbiome.

In adult dogs altered gut communities may also occur by drift over time or through larger scale shifts in composition due to environmental and diet factors. Infectious agents entering the digestive system or altered nutrients available to the microbial community due to diet changes can disrupt the balance of the microbiome leading to dysbiosis. Dysbiosis is described as an unbalancing of the microbial communities and, in the gut, can lead to clinical signs including gastrointestinal upset, diarrhoea, vomitting, nutritional deficiency and weight loss. Although the microbiome and microorganisms that exist within the gut are present in a continuum of abundance from shortly after birth or weaning until the late geriatric lifestages, an assault to the microbiome such as caused by infection, antibiotic clearance, medication or extreme diet change can alter the community composition throughout life. In cases where dogs have recurrent or chronic diarrhoea a characteristic pattern, signature or fingerprint in the microbiome may be detected and may indicate the likelihood of treatment being effective through dietary change and consequent changes in the microbiota.

The effect of nutrition on the microbiome is particularly suited to research in dogs and cats since complete and balanced diets may be fed as the sole source of food for extended periods of time, thus reducing confounding effects of dietary preference between individuals. That said, despite decades of research towards improving gastrointestinal (GI) health and optimizing faeces quality, there remains a very limited understanding of the bacterial taxa and functional gene groups associated with health parameters such as faecal form, apparent GI health (lack of clinical symptoms, resilience to challenge and recovery from infection) as well as GI dysfunction. This is particularly evident in hosts beyond the human such as dogs and cats.

Many ingredients are reported to alter host health and functional foods such as prebiotics and fibre are of particular use for gastrointestinal health and faeces quality or faecal consistency. In particular complex mixtures of soluble and insoluble fibre, probiotic oligosaccharides, glycans or beta-glucans can represent long recognised functional dietary ingredients that can be helpful for rectifying gut health conditions such as chronic and acute diarrhoea or bloating of the digestive system improving the health wellness and vitality of the host.

Studies assessing the effect of dietary fibre are particularly numerous since microbial populations in the large intestine selectively ferment digestion resistant fibre (De Godoy et al., 2013). As a result, there is a large body of data available from in vitro and in vivo testing of specific food ingredients including soluble and insoluble fibres and prebiotics. Such ingredients are also known to effect microbial populations within the intestinal tract and in particular within the colon differentially effecting populations that enhance health through fermentation of resistant fibre not digested by the host. Research to assess the effect of dietary fibre on the microbiota including soluble fibre, prebiotics and insoluble complex resistant dietary fibre considerd important for bulking properties and intestinal transit are numerous and have demonstrated effects both on faeces consistency and the microbial or microbially derived content of faeces (Ferrario et al., 2017; Wakshlag et al., 2011; Simpson et a1., 2001; Sunvold et al., 1995; Vickers et al., 2001).

Research findings can be difficult to compare in part due to the variation and complexity of many ingredients and fibre sources hence uncovering consistent trends remains problematic. Most naturally occurring dietary fibre sources represent mixtures of soluble and insoluble fibre with substantial research effort focusing on prebiotic oligosaccharides, glycans and beta-glucans. Specific product, inclusion level and host species are also confounding factors in the assessment of trends, however these ingredients have demonstrated effects both on faeces consistency and the microbial or microbially derived content of faeces (Ferrario et al., 2017; Wakshlag et al., 2011; Simpson et a1., 2001; Sunvold et al., 1995; Vickers et al., 2001). Although dosage and intake level are key to effectiveness, meta-analysis of human research studies on fibre and gastrointestinal health uncovered a relationship between fibre intake and incidence of colorectal cancer. High fibre intake although product / ingredient and inclusion level specific was described to reduce the risk of colorectal cancer in particular consumption of whole grains and cereal derived fibres were reported potentially protective.

The research literature describing fibre and prebiotic dietary supplementation in pet animals to date largely defines bacterial changes associated with particular ingredients measured by selective bacterial culture or now out-dated molecular techniques (such as denaturing gradient gel electrophoresis) often measuring those taxa known to be beneficial to human health. Whether these insights are valid across different hosts and are therefore relevant to canine health remains unclear. However, a small number of studies have been published that address changes in the microbiota as measured by deep sequencing upon feeding specific fibre sources and prebiotics in dogs. Middlebos et al., (2010) fed a diet containing 7.5% sugar beet pulp to dogs and found this amount to alter the structure of the gut microbiota detectably compared to the reference diet. Although the data supported only phylum level changes, significant increases in the Firmicutes and reductions in the Fusobacteria were observed when dogs received the diet containing sugar beet pulp.

In the manipulation of the faecal microbiota towards the enhancement of health, an understanding of the long term effects of dietary influence on the microbiome and the impact on host health parameters such as clinical biomarkers of health and gastrointestinal resilience and recovery would represent a significant advancement to this area of research.

Faeces consistency and particularly the extremes of loose or dry faeces are key indicators to owners of pet health and abhorrent faeces quality, diarrhoeic episodes and poor gastrointestinal healthis reportedly a major blocker to pet ownership, can have a significant effect on human animal interactions and hence can cause quality of life issues for people and their pet animals. As such, the impact of diet and ingredients on faeces quality in dogs and cats is of interest for the optimisation of pet health, wellness, lifestyle, vitality and nutrition. Dietary intake, including both dry matter volume, moisture and nutrient content can impact faeces consistency.

However, research on the gut microbiome and links with animal health is limited. Thus, there is a need for understanding the relationship between the gut microbiome and animal health wellness and vitaility. Measuring, monitoring and tracking of gut bacteria in faeces over time or with nutritional intake or intervention to enhance or support a healthy gut in pets would additionally be advantageous since this enable the pet owner to observe the effect of the composition, ingredient or nutritional factor and provides the impetuous for the pet owner to continue provisiding the health enhancing factor. Furthermore, there is a need for novel methods and compositions for enhancing gastrointestinal resilience, gut health in healthy pets and also for treating both chronic and acute intestinal disorders such as acute self limiting diarrhoeal infections and chronic enteropathies that are associated with the microbiome. Furthermore, there is a need to support gastrointestinal resilience following veterinary treatments and medicaments that effect the microbiome such as compositions ingredients and dietary boosters or toppers that reduce the occurance of microbiome dysbiosis and the resulting diarrhoea or soft faeces. Compositions or ingredients with such a utility would provide additional benefit aiding veterinarians in managing gastrointestinal symptoms of veterinary treatment.

Given the importance of the microbiome to health and wellbeing it is important to find ways to influence and to monitor and the status of the microbiome of an animal and to enable owners to observe the benefit through tracking health status over time, because of the inherent changes in the gut barrier, resilience to diarrhoea and gastrointestinal health that can occur with altered microbiome contents based on nutrient intake provided by owners and which impact on the health wellness and vitality of the pet.

### SUMMARY OF THE INVENTION

In some aspects, the present disclosure relates to compositions that change the microbiome of a companion animal, such as a canid, and methods comprising administering such compositions.

The compositions and methods disclosed herein influence, optimise and enhance a canid's gut microbiome and impacts gastrointestinal health and resilience and therein improving the health, wellness and vitality of the animal. Additionally, the present disclosure includes methods to monitor the microbiome through single and multi-point testing methods to enable the influence of the composition to be tested for and effect on the microbiome and as such to generate microbiome based gut health and resilience care pathways for enhancing pet wellness. The methods of the present disclosure can achieve this with high accuracy, as shown in the examples.

In a first aspect of the present disclsoure, the composition is suitable for a companion animal and includes at least 3 ingredients selected from: green tea polyphenols of about 0.005 grams/day to about 0.165 grams/day, wheat of about 0.5 grams/day to about 33 grams/day, cellulose of about 0.2 grams/day to about 30.8 grams/day, chicory pulp and/or beet pulp to a total amount of about 0.1 grams/day to about 11.0 g /day; tomato pomace (lycopene) of about 0.08 grams/day to about 2.2 grams/day, and fructooligosaccharides of about 0.025 grams/day to about 2.2 grams/day.

In one embodiment of the first aspect of the invention, the chicory pulp and/or beet pulp is present in a total amount of about 0.1 grams/day to about 8.0 g/day.

The composition may be a pet food, such as a nutritionally complete pet food, such as a dry (e.g., kibbles), semi-moist or moist pet food, a non-nutritionally complete pet food such as a supplement, functional topper, functional food booster, or nutraceutical or pharmaceutical composition.

In one embodiment, the composition further includes L-Camitine, fish oil, chondroitine sulfate, glucosamine, lutein, hydroxyproline, collagen. In a preferred embodiment, the further ingredients are relevant to the daily intake of the pet animal.

The ingredients can be included at concentration ranges around those shown in table 1.

**Table 1.**

| Ingredients | | |
|---|---|---|
| | Minimum | Maximum |
| | (grams/day) | (grams/day) |
| Green tea Polyphenols | 0.005 | 0.165 |
| L-Carnitine | 0.05 | 1.21 |
| Chondroitine sulfate and Glucosamine | 0.02 | 0.605 |
| Lutein | 0.00025 | 0.030 |
| Fish oil | 0.094 | 5.5 |
| Wheat | 0 | 16.5 |
| Cellulose Fibre 450 | 0.3 | 25 |
| Cellulose Fibre 600 | 0.1 | 25 |
| Sugar Beet Pulp | 0.05 | 11 |
| Chicory pulp | 0.05 | 4.4 |
| Tomato pomace | 0.025 | 2.2 |
| Fructooligosaccharides | 0.025 | 2.2 |
| | | |
| Dietary fiber (% dry matter in formulation) | 0.9 | 30.0 |
| Crude fat (% dry matter in formulation) | 1.4 | 19.8 |
| Protein (% dry matter in formulation) | 2.5 | 31.9 |
| | | |
| Collagen (% dry matter in formulation) | 0.05 | 11 |
| | | |
| Taurine (95% [w/w]) (% dry matter in formulation) | 0.0077 | 0.55 |
| | | |
| | | |
| OMEGA 6 | 2 | - |
| OMEGA 3 | 0.5 | - |
| EPA + DHA (50:50) | 0.025 | 0.55 |

in amounts that are around or within the minimum and/or maximum ranges specified.

In one embodiment, the composition includes all the ingredients within the ranges specified in Table 1.

In some aspects, the compositions are a nutritionally complete food, that is to say, the compositions provide all the nutrients necessary for a companion animal, without the need to supplement with other intake. An example would be a commercially produced pet food. Such a composition may have the nutrient profile of Table 2.

**Table 2.**

| Macronutrient | Min | Max |
|---|---|---|
| (% dry matter in formulation) | | |
| Dietary fiber | 7.5 | 19.8 |
| Crude fat | 10.0 | 19.8 |
| Protein | 2.5 | 44.0 |
| Indigestible protein | 0.08 | 4.4 |

In other aspects, the compsotions may be a non-nutrionally complete food such as a supplement, functional topper or functional food booster. Such compositions may have the nutrient profile as shown in Table 3.

**Table 3.**

| Macronutrient | Min | Max |
|---|---|---|
| (% dry matter in formulation) | | |
| DIETARY FIBER | 0.9 | 99.9 |
| CRUDE FAT | 1.0 | 19.8 |
| PROTEIN | 2.5 | 44.0 |
| Indigestible protein | 0.08 | 4.4 |

In certain embodiments, the ingredients in combination is at a concentration up to the maximum levels as described in Table 1.

In certain embodiments, the composition further includes an additional prebiotic. In certain embodiments, the composition further includes an additional fibre or other functional food ingredient.

In certain embodiments, the composition further contains a probiotic species of lactic acid bacterium such as a *Bifidobacterium, Lactobacilus* or *Entercoccus.* In certain embodiments, the composition further contains a probiotic species of yeast such as a species from the genus *Saccharomyces.* In certain embodiments, the composition further contains a spore forming probiotic bacterial species such as a species from the genus *Bacillus.*

In certain embodiments, the composition improves intestinal health in a companion animal within about 3 to about 21 days after administering the composition to the companion animal.

In certain embodiments, the composition is a dietary supplement In certain embodiments, the dietary supplement is added to the top of the pet food as a topper. In certain embodiments, the dietary supplement is subsequently mixed throughout the product. In certain embodiments, the composition is a dog food product.

The presently disclosed subject matter provides a method of improving intestinal health and resilience in a healthy companion animal or in an animal in need of improved gastrointestinal robustness such as an animal suffering acute or recurrent diarrhoea thereby improving resilience, health and wellness. In certain embodiments, the method includes administering to the companion animal an effective amount of any composition disclosed herein.

In another aspect of the present disclosure, there is provided a method of changing the microbiome of a companion animal by administering a composition as disclosed herein to a companion animal. In some embodiments, the method may comprise a first step of determining the health of the companion animal's microbiome, and a composition as disclosed herein is administered to the companion animal when there is determination of a healthy or an unhealthy microbiome detected in the first step, preferably an unhealthy microbiome.

The first step may include quantitating at least two, preferably at least three or at least four bacterial taxa in a sample obtained from the companion animal to determine their abundance; and comparing the determined abundance to the abundance of the same taxa in a control data set; wherein an increase or decrease in the abundance of the at least two, preferably at least three or at least four bacterial taxa relative to the control data set is indicative of a healthy or an unhealthy microbiome.

In another aspect of the present disclosure, the composition of the first aspect of the invention is used to increase the numbers of at least one, two, three, four, five, six or seven of *Faecalibacterium, Blautia, Allobaculum, Butyricicoccus, Slackia Lachnospira,* and Ruminococcaceae present in the gastro-intestinal tract or faeces of a companion animal compared to the number of said bacteria present in the companion animal before administration of the composition.

In still yet another aspect of the present disclosure, the compositions disclosed herein areused to decrease the numbers of at least one, two, three, four, five or six of Enterobacteriales, Escherichia, Enterobacteriaceae, Proteobacteria, Prevotella or Phascolarctobacterium present in the gastro-intestinal tract or faeces of a companion animal compared to the number of said bacteria present in the companion animal before administration of the composition.

In another ebodiment, the compositions disclosed herein are used to decrease the numbers of *Fusobacterium,* in particular *Fusobacterium mortiferum* or a species from the family Mogibacteriaceae or *Escherichia*/*Shigella* or Mediterraneibacter, or *Clostridium perfringens* or *Clostridium difficile.*

In yet another aspect of the present disclosure, the compositions disclosed herein are used to increase the gene expression of at least one of proline-, arginine-, alanine-, aspartic acid- and glutamic acid- related genes in a microbiome of a companion animal by administering the compositions to companion animal.

In yet another aspect of the present disclosure, the compositions are used to change the circulating amino acid levels in a companion animal. In one embodiment, the circulating amino acid levels of aspartic acid, serine, sarcosine, proline, glycine, a amino butyric acid, methionine, phenylalanine, 1-& 3-methylhistidine, carnosine, ornithine, and arginine are reduced by administering the compositions to the companion animal.

In another aspect of the present disclosure, the compositions disclosed herein are used to increase the CD3 and/or CD4 lymphocyte counts in a companion animal by administering the composition to the companion animal.

In another aspect of the present disclosure, the compositions disclosed herein are used to decrease the circulatory triglyceride levels in a companion animal by administering the compositions to the companion animal.

A healthy microbiome can be associated with reduced pathogen load, short chain fatty acid production and a reduced pH in the gut lumen is associated with reduced permeability of the gut barrier and gastrointestinal resilience. An unhealthy microbiome with pathogenic microorganisms represented at a higher bacterial load is associated with a number of health conditions. It is therefore desirable to monitor the health of the gut microbiome or to diagnose an unhealthy microbiome.

The health of a companion animal's microbiome can be measured by steps includingdetecting at least two, preferably at least three, preferably at least four bacterial taxa in a sample obtained from the companion animal; wherein the presence of the at least two, preferably at least three, preferably at least four bacterial taxa is indicative of an unhealthy microbiome.

The health of a companion animal's microbiome may also be determined by a method comprising the steps of calculating the diversity index for the species within the companion animal's microbiome and comparing the diversity index to the diversity index of a control data set.

The health of a companion animal may be determined by a method of the present disclosure on at least two time points. The time points may be between about 1 week, about 2 weeks, about 21 days, about 28 days, about 1 month, about 56 days, about 2 months, about 3 months, about 4 months, about 84 days, about 5 months or about 6 months apart. This is particularly useful where a companion animal is receiving treatment to shift the microbiome as it can monitor the progress of the therapy. It is also useful for monitoring the health of the companion animal. In one embodiment, the stability of the diversity index and/or the community composition of the microbiome is measured.

Also provided is a method of monitoring the health of the microbiome in a companion animal who has received thecompositions as disclosed herein. Such methods allow a skilled person to determine the success of the composition on the companion animal in shifting the microbiome. Preferably these methods comprise determining the health of the microbiome before and after treatment with the compositions disclosed herein as this helps to evaluate the success of the treatment.

The presently disclosed subject matter hereby provides a method by which health may be enhanced with receipt of a composition of dietary ingredients through a mainmeal or complementary pet care or pet food product in combination with methods for the determination of the gastrointestinal health of the animal, such that the owner or attending veterinarian is able to observe the impact on gastrointestinal health and resilience and thus is able to observe an effect of feeding composition on the animal and determine whether the companion animal will or has benefitted from an intervention to bring the microbiome back to its healthy state depending on the timing of the testing compared to feeding of the composition.

The presently disclosed subject matter additionally provides a method for assessing the intestinal health status in a healthy companion animal without signs of gastrointestinal upset and determine whether the companion animal will benefit from an intervention to bring the microbiome back to its healthy state. In certain embodiments, the presently disclosed subject matter provides a method for determining the intestinal health status in a companion animal in need thereof such as an animal with clinical signs such as diarrhoea or poor faeces quality or with intestinal dysbiosis such as chronic enteropathy or IBD.

In certain embodiments, the presently disclosed subject matter provides a method for determining the intestinal health status in a companion animal prior to receiving a pet care product such as a composition, such as a supplement a petfood functional topper or booster or a nutritionally complete dry kibble food thereby assessing the need for receiving the pet care product.

In certain embodiments, the presently disclosed subject matter provides a method for assessing the intestinal health status in a companion animal after receiving a pet care product such as a composition, such as a supplement a petfood functional topper or booster or a nutritionally complete dry kibble food thereby determining the gastrointestinal health of the animal after receipt of the product.

In certain embodiments, the presently disclosed subject matter provides a method for assessing the intestinal health status in a companion animal before, during and after receiving a pet care product such as a composition, such as a supplement a petfood functional topper or booster or a nutritionally complete dry kibble food thereby determining and monitoring gastrointestinal health of the animal before during and after receipt of the product such that the success of the pet care product can be assessed.

In certain embodiments, the method includes: a) measuring a first amount of a first intestinal microorganism and a second amount of a second intestinal microorganism in the companion animal; b) comparing the first amount of the intestinal microorganism with a first reference amount of the first intestinal microorganism, and comparing the second amount of the intestinal microorganism with a second reference amount of the second intestinal microorganism, wherein the reference amounts of the intestinal microorganisms are determined based on the amounts of the intestinal microorganisms in a plurality of healthy companion animals; and c) determining the intestinal health status in the companion animal when the first amount of the first intestinal microorganism is higher than the first reference amount of the first intestinal microorganism, and/or when the second amount of the second intestinal microorganism is lower than the second reference amount of the second intestinal microorganism.

In certain embodiments, the first intestinal microorganism is selected from the group comprising *Absiella*, *Anaerostipes, Anaerotruncus, Bacteroides plebeius*, *Bifidobacterium*, *Blautia, Butyricicoccus*, *Clostridium_sensu_stricto, Collinsella, Dorea, Enterococcus, Erysipelatoclostridium, Faecalibacterium. Finegoldia, Flavonifractur, Fusobacterium, Holdemania [Eubacterium] biforme, Lachnoclostridium, Lachnospiraceae_NK4A136_group, Lactobacillus, Megamonas, Pseudoflavomfractor, Romboutsia, Roseburia, Ruminococcaceae, Sellimonas sp., Terrisporobacter, Turicibacter* and *Lachnospiraceae.* Or from the genus *Fusobacterium*, in particular *Fusobacterium mortiferum* or a species from the family Mogibacteriaceae, or a species from the genera *Escherichia*/*Shigella* or *Clostridium perfringens* or *Clostridium difficile. Clostridium difficile* is used interchangeably with *Clostridium [Clostridioides]difficile* throughout.

In certain embodiments, the method further includes providing a customized recommendation of a treatment regimen, and/or further monitoring the intestinal microorganism, when the first amount of the first intestinal microorganism is lower than the first reference amount of the first intestinal microorganism, and/or when the second amount of the second intestinal microorganism is higher than the second reference amount of the second intestinal microorganism.

In certain embodiments, the amount of the intestinal bacterium is measured from a fecal sample of the subject.

The presently disclosed subject matter provides a method for treating an intestinal dysbiosis and/or improving intestinal health in a companion animal in need thereof. In certain embodiments, the method comprises: a) measuring a first amount of one or more intestinal or faecal microorganisms in the companion animal; b) administering a treatment regimen to the companion animal for treating the intestinal disorder and/or improving intestinal health; c) measuring a second amount of the intestinal microorganism in the subject after step b); and d) continuing administering the treatment regimen, when the second amount of the intestinal microorganism is changed compared to the first amount of the intestinal microorganism.

In certain embodiments, the intestinal microorganism is selected from *Absiella, Anaerostipes, Anaerotruncus, Bacteroides plebeius, Bifidobacterium, Blautia, Butyricicoccus, Clostridium_sensu_stricto, Collinsella, Dorea, Enterococcus, Erysipelatoclostridium, Faecalibacterium. Finegoldia, Flavonifractor, Fusobacterium, Holdemania [Eubacterium] biforme, Lachnoclostridium, Lachnospiraceae_NK4A136_group, Lactobacillus, Megamonas, Pseudoflavomfractor, Romboutsia, Roseburia, Ruminococcaceae, Sellimonas sp., Terrisporobacter, Turicibacter* and *Lachnospiraceae.* Or from the genus *Fusobacterium,* in particular *Fusobacterium mortiferum* or a species from the family Mogibacteriaceae, or a species from the genera *Escherichia*/*Shigella* or *Clostridium perfringens* or *Clostridium difficile* and any combination thereof. In some embodiments, the method includes continuing administering the treatment regimen, when the second amount of the intestinal microorganism is increased compared to the first amount of the intestinal microorganism. In certain embodiments, the intestinal microorganism is selected from the group consisting of *Faecalibacterium prausnitzii, Bacteroides plebeius, Holdemania [Eubacterium] biforme* and any combination thereof.

In certain embodiments, the intestinal microorganism is selected from *Absiella, Anaerostipes, Anaerotruncus, Bacteroides plebeius, Bifidobacterium, Blautia, Butyricicoccus, Clostridium _sensu_stricto, Collinsella, Dorea, Enterococcus, Erysipelatoclostridium, Faecalibacterium. Finegoldia, Flavonifractor, Fusobacterium, Holdemania [Eubacterium] biforme, Lachnoclostridium, Lachnospiraceae_NK4A136_group, Lactobacillus, Megamonas, Pseudoflavomfractor, Romboutsia, Roseburia, Ruminococcaceae, Sellimonas sp., Terrisporobacter, Turicibacter* and *Lachnospiraceae.* Or from the genus *Fusobacterium,* in particular *Fusobacterium mortiferum* or a species from the family Mogibacteriaceae, or a species from the genera *Escherichia*/*Shigella* or *Clostridium perfringens* or *Clostridium difficile* and any combination thereof. In some embodiments, the method further comprises continuing administering the treatment regimen, when the second amount of the intestinal microorganism is decreased compared to the first amount of the intestinal microorganism.

In certain embodiments, the second amount of the intestinal bacterium is measured between about 14 days or about 21 days or about 28 days or about 56 days or about 84 days after step b). In certain embodiments, the treatment regimen comprises a dietary regimen. In certain embodiments, the dietary regimen comprises administering an effective amount of any composition disclosed herein.

In certain embodiments, the amount of the intestinal microorganism is determined using a DNA sequencing technique.

In certain embodiments, the amount of the intestinal microorganism is determined using an RNA sequencing technique.

In certain embodiments, the amount of the intestinal microorganism is determined using a microarray.

In certain embodiments, the amount of the intestinal microorganism is determined using a polymerase chain reaction technique such as quantitative PCR

### BRIEF DESCRIPTION OF DRAWINGS

Figure 1. Figure 1 is a depiction of the interaction between intestinal bacterial flora and mucosal immunity in the intestinal mucosa.

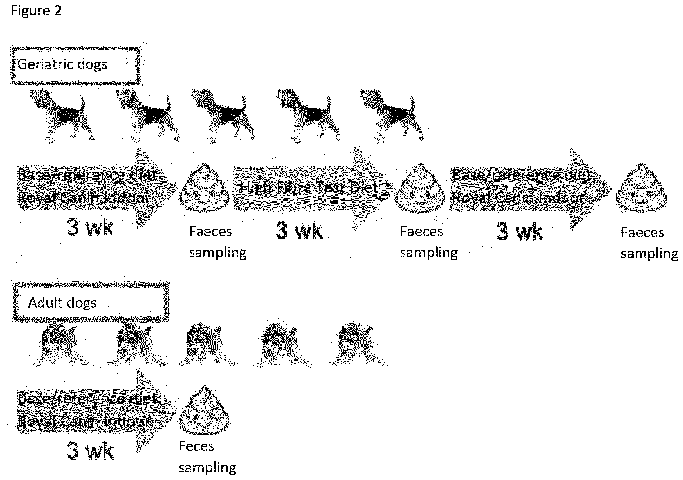

Figure 2. Figure 2 is a depiction of the feeding test study design as described in Example1.
Figure 3. Figure 3 is graphical depiction of the results of rarefaction analysis on DNA sequence data for detection of the gut microbiota from faeces samples as described in Example 1.
Figure 4. Figure 4 is a graphical depiction of the principal component analysis as described in Example 1.
Figure 5. Figure 5 is a graphical depiction of the comparison of the composition of intestinal bacterial flora between the adult and geriatric groups using LEfSe as described in Example 1.
Figure 6. Figure 6 is a graphical depiction of the comparison of changes in the composition of intestinal bacterial flora due to the influence of diet therapy in the geriatric group as described in Example 1.
Figure 7. Figure 7 is a histograms of functional genes in each group predicted using LEfSe as described in Example 1.
Figure 8. Figure 8 is a histograms of functional genes in each group predicted using LEfSe as described in Example 1.
Figure 9. Figure 9 is a graphical depiction of a Spider plot resulting from the Multiple factor analysis for visualisation of movements in the gross composition of the microbiota at 3 different days as described in Example 1.
Figure 10. Figure 10 is a graphical depiction of the PLS-DA correlation plot indicating correlations in the relative composition of the faecal microbiota based on 26 bacterial clusters (taxa) in 3 day pooled faeces samples from the 5 dogs individual dogs after 3 weeks of feeding different diets as described in Example 1.

### DISCLOSURE OF THE INVENTION

The present disclosure relates to dietary intervention methods for alteration of the gut microbiota in companion animals that impart a healthier status of the gut microbiome and additionally have determined methods for monitoring the influence of said dietary intervention on host health status.

In some aspects, the present disclosure is related to a composition including at least 3 ingredients selected from: green tea polyphenols of about 0.005 grams/day to about 0.165 grams/day, wheat of about 0.5 grams/day to about 33 grams/day, cellulose of about 0.2 grams/day to about 30.8 grams/day, chicory pulp and/or beet pulp to a total amount of about 0.1 grams/day to about 8.0 grams /day; tomato pomace (lycopene) of about 0.08 grams/day to about 2.2 grams/day, and fructooligosaccharides of about 0.025 grams/day to about 2.2 grams/day. In one embodiment of the first aspect of the invention, the composition comprises at least 3, 4, 5, 6 or all of the ingredients.

In one embodiment, the composition further comprises one or more of the ingredients selected from: L-Carnitine, fish oil, Chondroitine sulfate, Glucosamine, Lutein, hydroxyproline, collagen at levels relevant to the daily intake of an animal.

The ingredients of the disclosed compositions can be included at concentration ranges around those shown in table 1 in amounts that are at or within the minimum and/or maximum ranges specified.

In one embodiment, the composition comprises all the ingredients within the ranges specified in table 1.

The compositions disclosed herein may be a nutritionally complete food. As used herein, "nutritionally complete food" refers to a food that provides all the nutrients necessary for a companion animal, without the need to supplement with other intake. An example would be a commercially produced pet food. Such a composition may have the nutrient profile of Table 2.

In other aspects, the compositions may be a non-nutrionally complete food such as a supplement, functional topper or functional food booster. Such a composition may have the nutrient profile of Table 3.

In certain embodiments, the ingredients in combination is at a concentration up to the maximum levels as described in Table 1.

Specific ingredients are described that enable a shift in the microbial community composition reflective of enhanced health and resilience of the gut microbiome in dogs. Thus, ingredient combinations for administration to companion animals, e.g., dogs and cats, through pet food products, treatments, supplements, boosters or toppers are described along with methods of demonstrating, monitoring and tracking the effect of the composition on the individual pet.

Changes were observed in the microbiota consistent with an altered balance of the microbiota with an increased detection level of putatively health associated bacterial species and reduced levels of bacterial taxa associated with a less healthy microbiota (eg. opportunistic pathogens) when atest diet was fed to senior dogs compared to the baseline commercial dry pet food. The predicted microbiome functional content (by PICRUSt) suggested increased 'metabolism' and 'energy metabolism' in the microbiome when the animals received test diet compared to the standard base diet.

Systemic health was also altered on feeding the diet intervention described herein with significantly increased CD3+ and CD4+ lymphocyte counts and significantly lower circulating triglyceride levels observed when the test high fibre diet was fed to the dogs. Increased health associated bacteria were detected in the microbiome including, *Firecalibacterium, Blautia,* and *Lachnospira,* and significantldecreased Enterobacteriales, including *Escherichia* were detected in faeces and the functional gene composition was also altered with ether lipid metabolism-related genes decreased when dogs received the test diet while an increase in genes related to the metabolism of several amino acids was detected, including proline, arginine, alanine, aspartic acid, and glutamic acid.

Previous patent application (PCT US2020/014292 or WO2020/150712) which is herein incorporated in its entirety by reference identified thatbacterial species from certain bacterial taxa are indicative of a healthy or an unhealthy microbiome in canids, preferably dogs, and that therefore testing for the composition of the microbiota at multiple points before and after the intervention can allow the pet owner to understand the effect of the intervention on the pet. Whereingredients alter the composition of the gut microbiota such that an increase in abundance of the following organisms is imparted then the microbiome will be healthier leading to improved gastrointestinal resilience and may also impart systemic effects such as those observed on feeding the test diet herein: Microorganisms detected by the methods described therein indicative of the health of the canine gut microbiome include *Absiella, Anaerostipes, Allobaculum, Anaerotruncus, Bacteroides plebeius, Bifidobacterium, Blautia, Butyricicoccus, Clostridium_sensu*_*stricto, Collinsella, Dorea, Enterococcus, Erysipelatoclostridium, Faecalibacterium. Finegoldia, Flavonifractor, Fusobacterium, Holdemania [Eubacterium] biforme, Lachnoclostridium, Lachnospiraceae_NK4A136_group, Lactobacillus, Megamonas, Pseudoflavonifractor, Romboutsia, Roseburia, Ruminococcaceae, Sellimonas sp., Terrisporobacter, Turicibacter* and *Lachnospiraceae.* In contrast, a decrease in abundance is considered healthy for species from the genera *Fusobacterium,* in particular *Fusobacterium mortiferum* or a species from the family Mogibacteriaceae, or a species from the genera *Escherichia*/*Shigella* or *Clostridium perfringens* or *Clostridium difficile.*

The bacterial species may differ or the amounts may differ in what would be considered a healthy range depending on the life stage of the animal.

To date, there remains a need for novel methods and compositions for treating intestinal dysbiosis and other intestinal disorders that target the gut microbiome.

For clarity and not by way of limitation, the detailed description of the presently disclosed subject matter is divided into the following subsections:
1. Intestinal bacteria;
2. Compositions;
3. Treatment methods; and

### 1. INESTINAL BACTERIA

In certain embodiments, the intestinal microorganism can be used to indicate intestinal health in a subject. In certain embodiments, the intestinal microorganism is associated to a heathy status or an intestinal dysbiosis in a subject.

In certain embodiments, the intestinal microorganism indicates a healthy intestine status in a subject. In certain embodiments, the intestinal microorganism comprises a bacterium selected from the group consisting of *Lachnospiraceae sp., Faecalibacterium prausnitzii, Bacteroides plebeius, Holdemania [Eubacterium] biforme, Dorea sp., Ruminococcaceae sp., Bacteroides sp., Blautia sp., Erysipelotrichaceae sp., Lachnospiraceae sp.* and any combination thereof. In certain embodiments, the bacterium is selected from the group consisting of *Faecalibacterium prausnitzii, Bacteroides plebeius, Holdemania [Eubacterium] biforme* and any combination thereof.

### 2. COMPOSITIONS

In some aspects, the compositions described herein are suitable for a companion animal and comprise at least 3 ingredients selected from: green tea polyphenols of about 0.005 grams/day to about 0.165 grams/day, wheat of about 0.5 grams/day to about 33 grams/day, cellulose of about 0.2 grams/day to about 30.8 grams/day, chicory pulp and/or beet pulp to a total amount of about 0.1 grams/day to about 11.0 grams /day; tomato pomace (lycopene) of about 0.08 grams/day to about 2.2 grams/day, and fructooligosaccharides of about 0.025 grams/day to about 2.2 grams/day.

The composition may be a pet food, such as a nutritionally complete pet food, such as a dry (e.g., kibbles), semi-moist or moist pet food, a non-nutritionally complete pet food such as a supplement, functional topper, functional food booster, or nutraceutical or pharmaceutical composition.

In one embodiment, the composition further includes L-Carnitine, fish oil, Chondroitine sulfate, Glucosamine, Lutein, hydroxyproline, collagen at levels relevant to the daily intake of a pet animal.

In some aspects, the ingredients of compositions disclosed herien can be included at concentration ranges around those shown in Table 1.
in amounts that are at or within the minimum and/or maximum ranges specified.

In one embodiment, the composition comprises all the ingredients within the ranges specified in table 1.

In some aspects, the compositions may be a nutritionally complete food. As used herein, a "nutritionally complete food" is a food that provides all the nutrients necessary for a companion animal, without the need to supplement with other intake. An example would be a commercially produced pet food. Such a composition may have the nutrient profile of Table 2.

**Table 2.**

| Macronutrient | Min | Max |
|---|---|---|
| (% dry matter in formulation) | | |
| Dietary fiber | 7.5 | 19.8 |
| Crude fat | 10.0 | 19.8 |
| Protein | 2.5 | 44.0 |
| Indigestible protein | 0.08 | 4.4 |

In other aspects, the compositions may be a non-nutritionally complete food such as a supplement, functional topper or functional food booster. Such a composition may have the nutrient profile of Table 3.

**Table 3.**

| Macronutrient | Min | Max |
|---|---|---|
| (% dry matter in formulation) | | |
| DIETARY FIBER | 0.9 | 99.9 |
| CRUDE FAT | 1.0 | 19.8 |
| PROTEIN | 2.5 | 44.0 |
| Indigestible protein | 0.08 | 4.4 |

In certain embodiments, the ingredients in combination is at a concentration up to the maximum levels as described in Table 1. In some embodiments, the composition is a nutritionally complete pet food, such as a dry (e.g. kibbles), semi-moist or moist pet food, a non-nutritionally complete pet food such as a supplement, functional topper, functional food booster, or nutraceutical or pharmaceutical composition.

In one embodiment, such compositions are suitable for administration to a companion animal.

"Companion animal", as used herein, includes any animal that can be found in a domestic setting, including mammals such as canids (e.g., dogs and wolves) and felines (e.g., cats).

In one embodiment, the compositions described herein are suitable for use in a medicament.

In one embodiment, the compositions disclosed herein are suitable for use in treating gastro-intestinal dysbiosis in a companion animal. In one embodiment, the composition is suitable for use in altering the microbiome in a companion animal. In one embodiment, the composition is suitable for use to increase the numbers of Faecalibacterium, Blautia, Allobaculum, Butyricicoccus, Slackia Lachnospira, and Ruminococcaceae bacteria present in the gastro-intestinal tract or faeces of a companion animal compared to the number of said bacteria present in the companion animal before administration of the diet. In one embodiment, the composition is suitable for use to decrease the number of Enterobacteriales bacteria, preferably Escherichia, Enterobacteriaceae, Proteobacteria, Prevotella or Phascolarctobacterium. In one embodiment, the composition is for use to increase the gene expression of at least one of proline-, arginine-, alanine-, aspartic acid- and glutamic acid- related genes in a companion animal. In one embodiment, the composition is suitable for use to change the circulating amino acid levels in a companion animal, particularly reductions in any one of a group comprising aspartic acid, serine, sarcosine, proline, glycine, a amino butyric acid, methionine, phenylalanine, 1-& 3- methylhistidine, carnosine, ornithine, and arginine. In one embodiment, the composition is suitable for changing the circulating amino acid levels in a companion animal, particularly reducing any one of the amino acids in a group comprising aspartic acid, serine, sarcosine, proline, glycine, a amino butyric acid, methionine, phenylalanine, 1-& 3-methylhistidine, carnosine, ornithine, and arginine.

In certain embodiments, the compositions disclosed herein comprise an effective amount of pulp. The pulp is fibrous in nature. In certain embodiments, the pulp is beet pulp such as sugar beet pulp, preferably raw sugar beet pulp. In another embodiment, the pulp may be chicory pulp, preferably chicory pulp fibre. In certain embodiments, the pulp is cooked or sterilized or included with an extruded or a processed product. In one embodiment, the pulp of the composition may originate from more than one plant e.g. chicory and beet.

In certain embodiments, the pulp is at a concentration between about 0.5% w/w and about 10% w/w, between about 0.5% w/w and about 5% w/w, between about 0.5% w/w and about 4% w/w, between about 0.5% w/w and about 3% w/w, between about 0.5% w/w and about 2% w/w, between about 0.5% w/w and about 1.5% w/w, between about 0.5% w/w and about 1.2% w/w, between about 0.5% w/w and about 1% w/w, between about 0.5% w/w and about 0.9% w/w, or between about 0.5% w/w and about 0.8% w/w. In certain embodiments, the pulp is at a concentration between about 0.8% w/w and about 10% w/w, between about 0.8% w/w and about 5% w/w, between about 0.8% w/w and about 4% w/w, between about 0.8% w/w and about 3% w/w, between about 0.8% w/w and about 2% w/w, between about 0.8% w/w and about 1.5% w/w, between about 0.8% w/w and about 1% w/w, between about 1% w/w and about 10% w/w, between about 1% w/w and about 5% w/w, between about 2% w/w and about 5% w/w, or between about 1% w/w and about 2% w/w. In certain embodiments, the pulp is at a concentration of about 0.8% w/w.

In certain embodiments, the composition includes an effective amount of any bacterium disclosed herein that is associated to heathy intestine status in a subject. In certain embodiments, the bacterium is selected from the group consisting of Faecalibacterium, Blautia, Allobaculum, Butyricicoccus, Slackia Lachnospira, and Ruminococcaceae *Lachnospiraceae sp., Faecalibacterium prausnitzii, Bacteroides plebeius, Holdemania [Eubacterium] biforme, Dorea sp., Ruminococcaceae sp., Bacteroides sp., Blautia sp., Erysipelotrichaceae sp., Lachnospiraceae sp.* and any combination thereof. In certain embodiments, the bacterium is selected from the group consisting of *Faecalibacterium prausnitzii, Bacteroides plebeius, Holdemania [Eubacterium] biforme* and any combination thereof. In certain embodiments, the bacterium is selected from the group consisting of denovo1184, denovo1244, denovo1696, denovo2407, denovo2451, denovo283, denovo3487, denovo4154, denovo4328, denovo4681, denovo498, denovo5338, denovo6995, denovo943 and any combination thereof as defined in PCT US2020/014292/WO2020/150712 which is herein incorporated in its entirety by reference.

In certain embodiments, the bacterium included in the composition is between about 1 thousand CFU and about 10 trillion CFU. In certain embodiments, the bacterium is between about 1 thousand CFU and about 1 trillion CFU, between about 1 million CFU and about 1 trillion CFU, between about 100 million CFU and about 100 billion CFU, between about 1 billion CFU and about 1 trillion CFU, between about 1 billion CFU and about 100 billion CFU, between about 100 million CFU and about 100 billion CFU, between about 1 billion CFU and about 50 billion CFU, between about 100 million CFU and about 50 billion CFU, or between about 1 billion CFU and about 10 billion CFU. In certain embodiments, the bacterium comprised in the composition is at least about 1 thousand CFU, at least about 1 million CFU, at least about 10 million CFU, at least about 100 million CFU, at least about 1 billion CFU, at least about 10 billion CFU, at least about 100 billion CFU or more.

In certain embodiments, the composition further includes an effective amount of pulp such as chicory pulp or beet pulp (e.g., sugar beet pulp).

In certain embodiments, the composition is a dietary supplement for example applied on top of the composition, as a pet food topper or subsequently mixed throughout the product. In certain embodiments, the composition is a treat product or a chew or a kibble based treat or complementary product. In certain embodiments, the composition is a cat food product or a dog food product. In certain embodiments, the food product is a dog food product. In certain embodiments, the composition is a dry pet food product. In certain embodiments, the composition is a wet pet food product.

In certain embodiments, a formulation of the presently disclosed subject matter can further include an additional active agent. Non-limiting examples of additional active agents that can be present within a formulation of the presently disclosed subject matter include a nutritional agent (e.g., amino acids, peptides, proteins, fatty acids, carbohydrates, sugars, nucleic acids, nucleotides, vitamins, minerals, *etc*.), a probiotic, a probiotic, an antioxidant, and/or an agent that enhances the microbiome, improves gastrointestinal health and improves animal health.

In certain embodiments, the compositions include one or more probiotic. In certain embodiments, the probiotic is an animal probiotic. In certain embodiments, the animal probiotic is a feline probiotic. In certain embodiments, the animal probiotic is a canine probiotic. In certain embodiments, the probiotic is *Bifidobacterium, Lactobacillus, lactic acid bacterium* and/or *Enterococcus.* In certain embodiments, the probiotic is selected from any organism from lactic acid bacteria and more specifically from the following bacterial genera; *Lactococcus spp., Pediococcus spp., Bifidobacterium spp.* (*e.g., B. longum B. bifidum, B. pseudolongum, B. animalis, B infantis), Lactobacillus spp. (e.g. L. bulgaricus, L. acidophilus, L. brevis, Z casei, L. rhamnosus, L. plantarum, L. reuteri, L. fermentum, Enterococcus spp. (e.g. E. faecium), Prevotella spp., Fusobacterium spp, Alloprevotella spp*, and any combination thereof. In certain embodiments, the probiotic is administered to a companion animal in an amount of from about 1 colony forming unit (CFU) to about 100 billion CFUs per day for the maintenance of the GI microflora or the microbiome or gastrointestinal health. In certain embodiments, the probiotic is administered to a companion animal in an amount of from about 1 colony forming unit (CFU) to about 20 billion CFUs per day for the maintenance the GI microflora or the microbiome or gastrointestinal health. In certain embodiments, the probiotic is administered to a companion animal in an amount of from about 1 billion CFUs to about 20 billion CFUs per day for the maintenance of GI microflora. In certain embodiments, the probiotic is administered to a companion animal in amounts of from about 0.01 billion to about 100 billion live bacteria per day. In certain embodiments, the probiotic is administered to a companion animal in amounts of from about 0.1 billion to about 10 billion live bacteria per day. In certain embodiments, the probiotic is administered to a companion animal in amounts of from about 1x104 CFU to 1×10¹⁴CFU per day. In certain embodiments, an additional prebiotic can be included, such as fructooligosaccharides (FOS), xylooligosaccharides (XOS), galactooligosaccharides (GOS), glucans, galactans, arabinogalactan, inulin and/or mannooligosaccharides. In certain embodiments, the additional prebiotic is administered in amounts sufficient to positively stimulate the microbiome or the GI microflora and/or cause one or more probiotic to proliferate.

In certain embodiments, the composition can further contain additives known in the art. In certain embodiments, such additives are present in amounts that do not impair the purpose and effect provided by the presently disclosed subject matter. Examples of contemplated additives include, but are not limited to, substances that are functionally beneficial to improving health, substances with a stabilizing effect, organoleptic substances, processing aids, substances that enhance palatability, coloring substances, and substances that provide nutritional benefits. In certain embodiments, the stabilizing substances include, but are not limited to, substances that tend to increase the shelf life of the product. In certain embodiments, such substances include, but are not limited to, preservatives, synergists and sequestrants, packaging gases, stabilizers, emulsifiers, thickeners, gelling agents, and humectants. In certain embodiments, the emulsifiers and/or thickening agents include, for example, gelatin, cellulose ethers, starch, starch esters, starch ethers, and modified starches.

In certain embodiments, the additives for coloring, palatability, and nutritional purposes include, for example, colorants; iron oxide, sodium chloride, potassium citrate, potassium chloride, and other edible salts; vitamins; minerals; and flavoring. The amount of such additives in a product typically is up to about 5% (dry basis of the product).

In certain embodiments, the composition is a dietary supplement. In certain embodiments, the dietary supplements include, for example, a feed used with another feed to improve the nutritive balance or performance of the total. In certain embodiments, the supplements include compositions that are fed undiluted as a supplement to other feeds, offered free choice with other parts of an animal's ration that are separately available, or diluted and mixed with an animal's regular feed to produce a complete feed. The AAFCO, for example, provides a discussion relating to supplements in the American Feed Control Officials, Incorp. Official Publication, p. 220 (2003). Supplements can be in various forms including, for example, powders, liquids, syrups, pills, tablets, encapsulated compositions, *etc.*

In certain embodiments, the composition is a treat. In certain embodiments, treats include, for example, compositions that are given to an animal to entice the animal to eat during a non-meal time. In certain embodiments, the composition is a treat for canines include, for example, dog bones. Treats can be nutritional, wherein the product comprises one or more nutrients, and can, for example, have a composition as described above for food. Non-nutritional treats encompass any other treats that are nontoxic.

In certain embodiments, a bacterium and/or sugar beet pulp of the presently disclosed subject matter can be incorporated into the composition during the processing of the formulation, such as during and/or after mixing of other components of the product. Distribution of these components into the product can be accomplished by conventional means.

In certain embodiments, compositions of the presently disclosed subject matter can be prepared in a canned or wet form using conventional companion animal food processes. In certain embodiments, ground animal (e.g., mammal, poultry, and/or fish) proteinaceous tissues are mixed with the other ingredients, such as milk fish oils, cereal grains, other nutritionally balancing ingredients, special purpose additives (e.g., vitamin and mineral mixtures, inorganic salts, cellulose and beet pulp, bulking agents, and the like); and water that sufficient for processing is also added. These ingredients are mixed in a vessel suitable for heating while blending the components. Heating of the mixture can be effected using any suitable manner, such as, for example, by direct steam injection or by using a vessel fitted with a heat exchanger. Following the addition of the last ingredient, the mixture is heated to a temperature range of from about 50° F to about 212° F. Temperatures outside this range are acceptable but can be commercially impractical without use of other processing aids. When heated to the appropriate temperature, the material will typically be in the form of a thick liquid. The thick liquid is filled into cans. A lid is applied, and the container is hermetically sealed. The sealed can is then placed into conventional equipment designed to sterilize the contents. This is usually accomplished by heating to temperatures of greater than about 230° F for an appropriate time, which is dependent on, for example, the temperature used and the composition.

In certain embodiments, the composition of the presently disclosed subject matter can be prepared in a dry form using conventional processes. In certain embodiments, dry ingredients, including, for example, animal protein sources, plant protein sources, grains, *etc*., are ground and mixed together. In certain embodiments, moist or liquid ingredients, including fats, oils, animal protein sources, water, *etc*., are then added to and mixed with the dry mix. In certain embodiments, the mixture is then processed into kibbles or similar dry pieces. In certain embodiments, composition is a kibble. In certain embodiments, kibble is formed using an extrusion process in which the mixture of dry and wet ingredients is subjected to mechanical work at a high pressure and temperature and forced through small openings and cut off into kibble by a rotating knife. In certain embodiments, the wet kibble is then dried and optionally coated with one or more topical coatings which can include, for example, flavors, fats, oils, powders, and the like. In certain embodiments, kibble can also be made from the dough using a baking process, rather than extrusion, wherein the dough is placed into a mold before dry-heat processing.

In certain embodiments, treats of the presently disclosed subject matter can be prepared by, for example, an extrusion or baking process similar to those described above for dry food.

### 3. TREATMENT METHODS

In certain non-limiting embodiments, the presently disclosed subject matter provides methods for enhancing or improving the microbiome, for improving intestinal health and/or treating an intestinal dysbiosis of a subject in need thereof. In certain embodiments, the subject is a companion animal, e.g., a dog or a cat. In certain embodiments, the method can improve immunity, digestive function and/or reduce dysbiosis of a companion animal.

In certain embodiments, the method comprises administering to the subject an effective amount of any presently disclosed compositions. In certain embodiments, the method further comprises monitoring any presently disclosed intestinal microorganism in the subject. In certain embodiments, the intestinal microorganism is measured in a fecal sample of the subject. In certain embodiments, the intestinal microorganism is measured in a sample from the intestines of the subject.

In certain embodiments, the composition can be administered to a subject from 20 times per day to once per day, from 10 times per day to once per day, or from 5 times per day to once per day. In certain embodiments, the composition can be administered to a subject once per day, twice per day, thrice per day, 4 times per day, 5 times per day, 6 times per day, 7 times per day, 8 times per day, 9 times per day, 10 or more times per day. In certain embodiments, the composition can be administered to a subject once per two days, once per three days, once per four days, once per five days, once per six days, once a week, once per two weeks, once per three weeks, or once per month. In certain embodiments, the composition can be administered to an animal in a constant manner, *e.g*., where the animal grazes on a constantly available supply of the subject composition.

In certain embodiments, the dosage of the composition is between about 1 mg/kg body weight per day and about 5000 mg/kg body weight per day. In certain embodiments, the dosage of the pet food product is between about 5 mg/kg body weight per day and about 1000 mg/kg body weight per day, between about 10 mg/kg body weight per day and about 500 mg/kg body weight per day, between about 10 mg/kg body weight per day and about 250 mg/kg body weight per day, between about 10 mg/kg body weight per day and about 200 mg/kg body weight per day, between about 20 mg/kg body weight per day and about 100 mg/kg body weight per day, between about 20 mg/kg body weight per day and about 50 mg/kg body weight per day or any intermediate range thereof. In certain embodiments, the dosage of the pet food product is at least about 1 mg/kg body weight per day, at least about 5 mg/kg body weight per day, at least about 10 mg/kg body weight per day, at least about 20 mg/kg body weight per day, at least about 50 mg/kg body weight per day, at least about 100 mg/kg body weight per day, at least about 200 mg/kg body weight per day or more. In certain embodiments, the dosage of the pet food product is no more than about 5 mg/kg body weight per day, no more than about 10 mg/kg body weight per day, no more than about 20 mg/kg body weight per day, no more than about 50 mg/kg body weight per day, no more than about 100 mg/kg body weight per day, no more than about 200 mg/kg body weight per day, no more than about 500 mg/kg body weight per day or more.

In certain embodiments, the amount of composition decreases over the course of feeding a companion animal. In certain embodiments, the concentration of the composition increases over the course of feeding a companion animal. In certain embodiments, the concentration of the composition is modified based on the age of the companion animal.

In certain non-limiting embodiments, the presently disclosed subject matter provides for a method for treating an intestinal dysbiosis and/or improving intestinal health in a companion animal in need thereof. In certain embodiments, the method comprises: a) measuring a first amount of one or more intestinal microorganism in the companion animal; b) administering the composition of the present disclosure to the companion animal for treating the intestinal disorder and/or improving intestinal health; c) measuring a second amount of the intestinal microorganism in the subject after step b); and d) continuing administering the composition of the present disclosure, when the second amount of the intestinal microorganism is changed compared to the first amount of the intestinal microorganism.

In certain embodiments, the intestinal microorganism is selected from denovo1184, denovo1244, denovo1696, denovo2407, denovo2451, denovo283, denovo3487, denovo4154, denovo4328, denovo4681, denovo498, denovo5338, denovo6995, denovo943 (as defined in PCT US2020/014292 or WO2020/150712) and any combination thereof, and wherein step d) includes continuing administering the treatment regimen, when the second amount of the intestinal microorganism is increased compared to the first amount of the intestinal microorganism. In certain embodiments, the intestinal microorganism is selected from the group consisting of *Faecalibacterium prausnilzii, Bacteroides plebeius, Holdemania [Eubacterium] biforme* and any combination thereof.

In certain embodiments, the second amount of the intestinal microorganism is measured between about 7 days and about 14 days after step b). In certain embodiments, an amount of the intestinal microorganism is increased within about 21 days, within about 14 days, within about 12 days, within about 10 days, within about 7 days, within about 6 days, within about 5 days, within about 4 days, within about 3 days, within about 2 days, or within about 1 day after step b). In certain embodiments, an amount of the intestinal bacterium is increased within about 1 day to about 21 days, within about 1 days to about 14 days, within about 3 days to about 14 days, within about 5 days to about 14 days, within about 7 days to about 14 days, within about 10 days to about 14 days, or within about 7 days to about 21 days after step b).

In certain embodiments, the intestinal microorganism is selected from denovo1214, denovo1400, denovo1762, denovo2014, denovo2197, denovo2368, denovo3663, denovo4206, denovo4485, denovo6368, denovo7117, denovo4881 and any combination thereof, and wherein step d) comprises continuing administering the composition, when the second amount of the intestinal microorganism is decreased compared to the first amount of the intestinal microorganism.

In certain embodiments, the second amount of the intestinal microorganism is measured between about 7 days and about 14 days after step b). In certain embodiments, an amount of the intestinal microorganism is decreased within about 21 days, within about 14 days, within about 12 days, within about 10 days, within about 7 days, within about 6 days, within about 5 days, within about 4 days, within about 3 days, within about 2 days, or within about 1 day after step b). In certain embodiments, an amount of the intestinal bacterium is decreased within about 1 day to about 21 days, within about 1 days to about 14 days, within about 3 days to about 14 days, within about 5 days to about 14 days, within about 7 days to about 14 days, within about 10 days to about 14 days, or within about 7 days to about 21 days after step b).

In certain embodiments, the reference amount of an intestinal microorganism is a mean amount of the intestinal microorganism in a plurality of healthy companion animals. In certain embodiments, the reference amount of an intestinal microorganism is within about three standard deviations of a mean amount of the intestinal microorganism in a plurality of healthy companion animals. In certain embodiments, the reference amount of an intestinal microorganism is within about two standard deviations of a mean amount of the intestinal microorganism in a plurality of healthy companion animals. In certain embodiments, the reference amount of an intestinal microorganism is within about one standard deviation of a mean amount of the intestinal microorganism in a plurality of healthy companion animals.

In certain embodiments, the amount of an intestinal microorganism can be determined by any method known in the art. In certain embodiments, the method includes, but is not limited to, antibody-based detection methods detecting a protein/antigen associated with the microorganism, e.g., an enzyme-linked immunosorbent assay (ELISA), flow cytometry, western blot; and methods for detecting a 16s rRNA associated with the microorganism, e.g., real-time polymerase chain reaction (RT-PCR), quantitative polymerase chain reaction (qPCR), DNA sequencing and microarray analyses. In certain embodiments, the microarray comprises probes for detecting any of the intestinal microorganism disclosed herein.

In certain embodiments, the treatment regimen can be any treatment regimen of dysbiosis known in the art. In certain embodiments, the treatment regimen comprises a treatment method disclosed herein.

In certain embodiments, the amount of the intestinal bacterium is measured from a fecal sample of the subject.

### The control data set

In some embodiments, the abundance of the bacterial species is compared to a control data set from a canid with a similar chronological age, e.g. a puppy, an adult canid, a senior canid or a geriatric canid.

Alternatively, or in addition, a control data set may be prepared. To this end the microbiome of two or more (e.g. 3, 4, 5, 10, 15, 20 or more) healthy canids may be analysed for the abundance of the species contained in the microbiome. A healthy canid in this context is a canid who has not been diagnosed with a disease that is known to affect the microbiome. Examples of such diseases include irritable bowel syndrome, ulcerative colitis, Crohn's and inflammatory bowel disease. Preferably, the canid does not suffer from dysbiosis. Dysbiosis refers to a microbiome imbalance inside the body, resulting from an insufficient level of keystone bacteria (e.g., *bifidobacteria*, such as *B. longum subsp. infantis*) or an overabundance of harmful bacteria in the gut. Methods for detecting dysbiosis are well known in the art. The two or more canids will generally be from a particular life stage. For example, they may be puppies, adult canids, senior canids or geriatric canids. This is useful because the microbiome changes in a canid's lifetime and the microbiome therefore needs to be compared to a canid at the same lifestage. Where the canid is a dog, the control data set may further be from a dog of the same breed or, where the dog is a mongrel, the same breed as one of the direct ancestors (parents or grandparents) of the dog.

Specific steps to prepare the control data set may comprise analysing the microbiome composition of at least two (*e.g.* 3, 4, 5, 6, 7, 8 9, 10, 15, 20 or more) puppies, and/or at least two (*e.g.* 3, 4, 5, 6, 7, 8 9, 10, 15, 20 or more) adult canids, and/or at least two (*e.g*. 3, 4, 5, 6, 7, 8 9, 10, 15, 20 or more) senior canids and/or at least two (*e.g.* 3, 4, 5, 6, 7, 8 9, 10, 15, 20 or more) geriatric canids; determining the abundance of bacterial species (in particular those discussed above); and compiling these data into a control data set.

For embodiments where the diversity index of the microbiome is determined, the control data set may be prepared in a similar manner. In particular, the diversity index can be determined in two or more healthy canids at a particular life stage (puppy, adult, senior or geriatric). The results can then be used to identify the mean range for the diversity index in a canid at that life stage.

It will be understood that the control data set does not need to be prepared every time the methods disclosed herein are performed. Instead, a skilled person can rely on an established control set.

Techniques which allow a skilled person to detect and quantitate bacterial taxa are well known in the art. These include, for example, 16S rDNA amplicon sequencing, shotgun sequencing, metagenome sequencing, Illumina sequencing, and nanopore sequencing. Preferably, the bacterial taxa are determined by sequencing the 16s rDNA sequence.

In some embodiments, the bacterial taxa are determined by sequencing the V4-V6 region, for example using Illumina sequencing. These methods may use the primers 319F: and 806R as described in PCT US2020/014292 or WO2020/150712.

The bacterial species may also be detected by other means known in the art such as, for example, RNA sequencing, protein sequence homology or other biological marker indicative of the bacterial species.

The sequencing data can then be used to determine the presence or absence of different bacterial taxa in the sample. For example, the sequences can be clustered at 98%, 99% or 100% identity and abundant taxa (e.g. those representing more than 0.001 of the total sequences) can then be assessed for their relative proportions. Suitable techniques are known in the art and include, for example, logistic regression, partial least squares discriminate analysis (PLS-DA) or random forest analysis and other multivariate method.

### The canid

The methods disclosed herein can be used to determine the microbiome health of a canid. This genus comprises domestic dogs (*Canis lupus familiaris*), wolves, coyotes, foxes, jackals, dingoes and the invention can be used for all these animals. Most preferably, the subject is a domestic dog, herein referred to simply as a dog.

The canid may be healthy. "Healthy" may refer to a canid who has not been diagnosed with a disease that is known to affect the microbiome. Examples of such diseases include irritable bowel syndrome, ulcerative colitis, Crohn's and inflammatory bowel disease. Preferably, the canid does not suffer from dysbiosis. Dysbiosis refers to a microbiome imbalance inside the body, resulting from an insufficient level of keystone bacteria (e.g., *bifidobacteria*, such as *B. longum subsp. infantis*) or an overabundance of harmful bacteria in the gut. Methods for detecting dysbiosis are well known in the art.

One advantage of the methods disclosed herein is that they allow a skilled person to determine whether the canid's microbiome is healthy, taking into account the canid's lifestage.

There are numerous different breeds of domestic dogs which show a diverse habitus. Different breeds also have different life expectancies with smaller dogs generally being expected to live longer than bigger breeds. Accordingly, different breeds are considered to be puppies, adult, senior or geriatric at different time points in their life. A summary of the different life stages is provided in the table below.

**Table 4.**

| | Youth | Adult | Senior | Geriatric |
|---|---|---|---|---|
| Toy | Up to 7 years | 8-11 years | 12-13 years | 14+years |
| Small | Up to 7 years | 8-11 years | 12-13 years | 14+years |
| Medium | Up to 5 years | 6-9 years | 10-13 years | 14+years |
| Large | Up to 5 years | 6-9 years | 10-11 years | 12+years |

The distinction between toy, small, medium and large breeds is known in the art. In particular, toy breeds comprise distinct breeds such as Affenpinscher, Australian Silky Terrier, Bichon Frise, Bolognese, Cavalier King Charles Spaniel, Chihuahua, Chinese Crested, Coton De Tulear, English Toy Terrier, Griffon Bruxellois, Havanese, Italian Greyhound, Japanese Chin, King Charles Spaniel, Lowchen (Little Lion Dog), Maltese, Miniature Pinscher, Papillon, Pekingese, Pomeranian, Pug, Russian Toy and Yorkshire Terrier.

Small breeds are larger on average than toy breeds with an average body weight of up to about 10kg. Exemplary breeds include French Bulldog, Beagle, Dachshund, Pembroke Welsh Corgi, Miniature Schnautzer, Cavalier King Charles Spaniel, Shih Tzu, and Boston Terrier.

Medium dog breeds have an average weight of about 11-26kg. These dog breeds include Bulldog, Cocker Spaniel, Shetland Sheepdog, Border Collie, Basset Hound, Siberian Husky and Dalmatian.

Large breed are those with an average body weight of at least 27kg. Examples include Great Dane, Neapolitan mastiff, Scottish Deerhound, Dogue de Bordeaux, Newfoundland, English mastiff, Saint Bernard, Leonberger and Irish Wolfhound.

Cross-breeds can generally be categorised into toy, small, medium and large dogs depending on their body weight.

### The sample

The methods disclosed herein generally use a fecal sample or a sample from the gastrointestinal lumen of the canid. Fecal samples are convenient because their collection is non-invasive and it also allows for easy repeated sampling of individuals over a period of time. The invention can also be used with other samples, such as ileal, jejunal, duodenal samples and colonic samples.

The sample may be a fresh sample. The sample may also be frozen or stabilised by other means such as addition to preservation buffers or by dehydration using methods such as freeze drying before use in the methods of the invention.

Before use in the methods disclosed herein, the sample will generally be processed to extract DNA. Methods for isolating DNA are well known in the art, as reviewed in reference Hart et al. (2015) PLoS One. Nov 24;10(11):e0143334, for example. Suitable methods include, for instance, the QIAamp Power Faecal DNA kit (Qiagen).

### Changing the microbiome

In some embodiments, the methods may comprise a further step of changing the composition of the microbiome. This can be achieved by administering a dietary change, a functional food or nutraceutical, a pharmaceutical composition which is able to change the composition of the microbiome. Such functional foods, nutraceuticals, live biotherapeutic products (LBPs) and pharmaceutical compositions are well known in the art and comprise bacteria (see WO2018/006080). They may comprise single bacterial species selected from *Bifidobacterium* sp. such as *B. animalis (e.g., B. animalis subsp. animalis or B. animalis subsp. lactis], B. bifidum, B. breve, B. longum (e.g., B. longum subsp. infantis or B. longum subsp. longum), B. pseudolongum, B.adolescentis, B. catenulatum,or B. pseudocatanulatum,* single bacterial species of *Lactobacillus, such as L. acidophilus, L. antri, L. brevis, L. casei, L. coleohominis, L. crispatus, L. curvatus, L. fermentum, L. gasseri, L. johnsonii, L. mucosae, L. pentosus, L. plantarum, L. reuteri, L. rhamnosus, L. sakei, L. salivarius, L. paracasei, L. kisonensis., L. paralimentarius, L. perolens, L. apis, L. ghanensis, L. dextrinicus, L. shenzenensis, L. harbinensis,* or single bacterial species of *Pediococcus, such as P. parvulus, P. lolii, P. acidilaclici, P. argentinicus, P. claussenii, P. pentosaceus,* or *P. stilesii or similarly species of Enterococcus such as E. faecium or Bacillus* species such as *Bacillus subtilis, B. coagulans B. indicus or B. clausii.* Additionally, they may include combinations of these and other bacterial species.

These methods may be useful where a canid's microbiome age does not positively concur with its actual age. For example, the methods disclosed herein may reveal that an adult dog has a microbiome composition and diversity representative of a senior or geriatric dog. As discussed above, characteristics associated with the adult microbiome are considered the healthiest microbiome characteristics and so in these circumstances it would be highly desirable in the older dog to make a dietary change and/or to administer a functional food, nutraceutical, or pharmaceutical composition to shift the microbiome back to an adult microbiome composition/status.

Similarly, it may be desirable to shift the microbiome so that the microbiome biological age status does not concur with the canid's actual age. For example, an older dog in the senior or geriatric life stage suffering from recurrent diarrhea may benefit from receiving a diet change, functional food, nutraceutical, LBP or pharmaceutical composition to shift the microbiome to one representative of an adult dog.

In some aspects, the methods disclosed herein may also be used to assess the success of a treatment as described above. To this end a canid may receive a change in diet, functional food, supplement, LBP, nutraceutical or pharmaceutical composition or an exercise/physical activity regimen, which is capable of changing the composition of the microbiome. Following administration of the treatment through nutrition or exercise (for example after 1 day, 2 days, 5 days, 1 week, 2 weeks, 3 weeks, 1 month, 3 months, 6 months etc.), the microbiome age may be assessed using any of the methods of the invention. Preferably, the microbiome biological age status is determined before and after administration of the dietary change, functional food, supplement, LBP, nutraceutical or pharmaceutical composition or exercise/physical activity regimen.

### Monitoring

In some aspects, the compositions disclosed hereinmay be fed to a companion animal who is monitored. The first time the method is performed the microbiome age of the companion animal is determined and, following administration of a composition disclosed herein the method is repeated to assess the influence of the composition. The microbiome biological age status may also be determined for the first time after the canid has received treatment and the method repeated afterwards to assess whether there is a change in the microbiome biological age status.

The method may be repeated several days, one week, two weeks, three weeks, one month, two months, three months, four months, five months, six months, 12 months, 18 months, 24 months, 30 months, 36 months, or more than 36 months apart.

### General

As used herein, the terms "comprises," "comprising," or any other variation thereof, are intended to cover a non-exclusive inclusion, such that a process, method, article, or apparatus that comprises a list of elements does not include only those elements but can include other elements not expressly listed or inherent to such process, method, article, or apparatus.

The term "about" in relation to a numerical value *x* is optional and means, for example, *x*±10%.

The word "substantially" does not exclude "completely" *e.g.* a composition which is "substantially free" from Y may be completely free from Y. Where necessary, the word "substantially" may be omitted from the definition of the invention.

References to a percentage sequence identity between two nucleotide sequences means that, when aligned, that percentage of nucleotides are the same in comparing the two sequences. This alignment and the percent homology or sequence identity can be determined using software programs known in the art. A preferred alignment is determined using the BLAST (basic local alignment search tool) algorithm or the Smith-Waterman homology search algorithm using an affine gap search with a gap open penalty of 12 and a gap extension penalty of 2, BLOSUM matrix of 62. The alignment may be over the entire reference sequence, *i.e.* it may be over 100% length of the sequences disclosed herein.

Unless specifically stated, a process or method comprising numerous steps may comprise additional steps at the beginning or end of the method, or may comprise additional intervening steps. Also, steps may be combined, omitted or performed in an alternative order, if appropriate.

Various embodiments of the invention are described herein. It will be appreciated that the features specified in each embodiment may be combined with other specified features, to provide further embodiments. In particular, embodiments highlighted herein as being suitable, typical or preferred may be combined with each other (except when they are mutually exclusive).

### MODES FOR CARRYING OUT THE INVENTION

### Example 1: A method of influencing and thereby improving or enhancing the gut microbiome or microbiota of dogs

The inventors performed an investigation to study how the composition of intestinal bacterial flora and amino acid metabolic function changed in geriatric dogs and additionally compared the geriatric microbiota and microbiome (via PICRUST) with that of adult dogs. In the present study, changes in intestinal bacterial flora in older dogs (e.g. geriatric dogs) fed a therapeutic diet were examined and compared with the intestinal bacterial flora in adult dogs.

### Materials and Methods

Ten beagle dogs were involved in the study. None of the dogs received medication for 3 months before the study. They were clinically asymptomatic, and the absence of digestive disease was confirmed based on CBC, serum biochemistry, urinalysis, fecal test, and abdominal ultrasonography. Five dogs were included in the geriatric and adult dog groups, respectively.

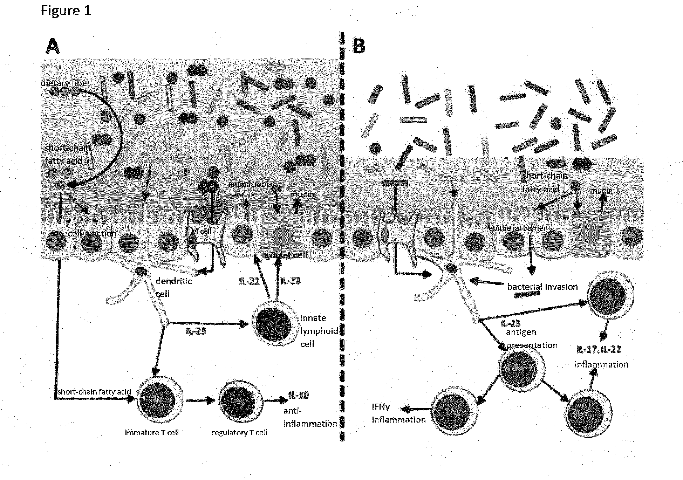

Each dog was fed a commercially available maintenance diet for adult dogs for 3 weeks as a stabilisation and introduction period. Then, the 5 dogs in the geriatric group were fed a diet of a different formulation containing a higher level of dietary fibre imparted through a blend of complex insoluble fibre and soluble fibres including short chain prebiotics for 3 weeks, the animals then returned to the base maintenance diet for a further 3 weeks (Fig. 1). Testing of blood haematology and biochemistry was performed and serum was stored at -80°C at each time point of switching the dogs to the alternative diet (day 0; ie after 21 days on base diet, day 21; ie 21 days after switching to the test formulation diet, and day 42 ie after a further 21 days following the return to base diet). Faeces were manually collected and immediately stored at -80°C for 3 consecutive days, and subsequently subjected to analysis.

At each sampling time point, CBC, serum biochemistry, differential white blood count, and lymphocyte subsets were measured and the frozen serum samples were measured for amino acid fractions.

The fecal samples collected for 3 consecutive days were pooled and 16S analysis of the 16S rRNA gene V3-V4 region using Illumina MiSeq and QIIME pipeline software ver. 1.8, and for analysis of bacterial flora composition in each sample was carried out. Furthermore, predictive analysis of functional gene composition was performed based on KEGG Orthology using PICRUSt.

Differences between the groups in age, sex, body weight, diversity score of intestinal bacterial flora, and serum amino acid fractional concentrations were analyzed using Fisher's exact test or Dunnett's test, and test diet-induced changes in the geriatric group were analyzed using the Kruskal-Wallis test and Friedman test (StatMate III, ATMS). Similarity of intestinal bacterial flora composition between the groups was performed using Analysis of Similarity (ANOSIM, PRIMER 6), and differences in the composition ratios of each bacterial species and predicted functional gene were analyzed using the linear discriminant analysis effect size method (LEfSe: https://huttenhower.sph.harvard.edu/galaxy/).

### Results

The sex and body weight in the 2 groups are shown in Table 6. No significant difference was noted between the 2 groups. No digestive symptom was observed in any animal throughout the test diet feeding period.

On blood tests, WBC, Lym, and Mon were significantly lower in the geriatric group than in the adult group, and notably the CD3+, CD4+, CD8+, and Foxp3+ lymphocyte counts were significantly lower (Table 7). In addition, when the dogs received the test high fibre diet significantly increased CD3+ and CD4+ lymphocyte counts were observed, but no change was noted in the Foxp3+ lymphocyte count. The TG level was significantly higher in the geriatric group, and this was improved by the diet for elderly dogs.

When the serum amino acid fractions were compared, the serine, glycine, and carnosine levels were significantly lower in the geriatric group, and these were not markedly changed by changing diet (Table 8). The lysine level was however significantly higher in the geriatric group and a relationship was observed with diet. Feeding of the high fibre test diet to the dogs was associatd with a reduction to a level comparable with that in the adult group. In addition, the high fibre test diet slightly reduced several amino acids such as aspartic acid, sarcosine, proline, α-amino butyric acid, methionine, phenylalanine, 1-methylhistidine, 3-methylhistidine, ornithine, and arginine.

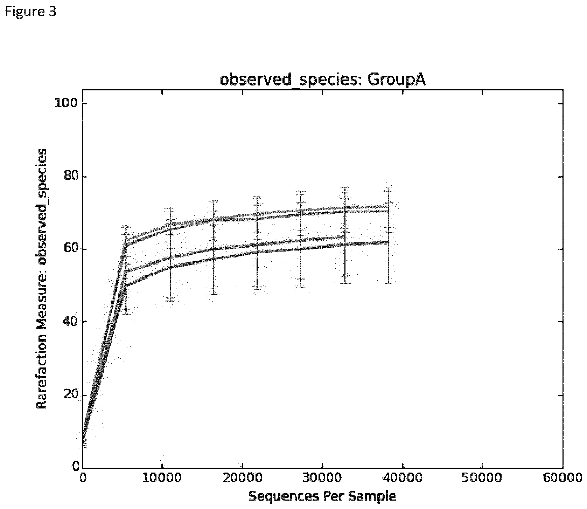

On 16S analysis, 215,394 sequence leads were acquired per sample on average (median: 221,640, range: 164,416-250,500). Rarefaction analysis was performed to avoid an influence of differences in the sequence depth among samples (Figure 3). As a specific plateau was reached in all samples when more than 10,000 leads were extracted, 10,000 leads were randomly extracted from each sample in the analyses below. A curve was prepared for each group. Red: Adult group, orange: geriatric group (day 0) (th, green: geriatric group (day 21), blue: geriatric group (day 42). Data are presented as the mean ± standard deviation. In order, when looking at the 30000 sequences per sample mark, the curves from highest to lowest value rarefaction measures: orange, green, red, then blue.

To compare the diversity of intestinal bacterial flora, the number of detected OTU, and the PD, Chaol, and Shannon indices were calculated. No significant differences were noted in any index of microbial diversity between the groups or diet intervention (Table 9).

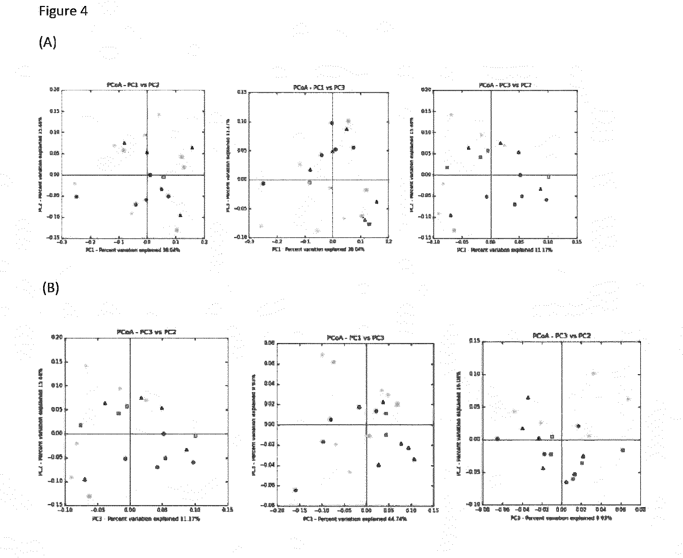

In unweighted unifrac distance-based principal component analysis, no significant difference was noted in the composition of bacterial species between the groups of dogs (ANOSIM; Global R = 0.095, P = 0.094) (Fig.4A). Weighted unifrac distance-based principal component analysis determined that the geriatric dogs had a significantly different composition of bacterial flora on day 21 after receiving the high fibre test diet for a period of 3 weeks compared with that on day 42 after the return to the base diet and that in the adult group (ANOSIM; Global R = 0.203, *P* = 0.050; day 21 vs. day 42, R = 0.312, *P* = 0.024; day 21 vs. Adult, R = 0.448, *P* = 0.008), but the bacterial flora composition in the geriatric group on day 0 was not significantly different from that on day 21 or that in the adult group (day 0 vs. Adult, R = 0.168, *P* = 0.079; day 0 vs. day 21, R = -0.016, *P* = 0.540) (Fig. 4B).

The graphs for Figure 4 were prepared based on the unweighted unifrac distance (A) and weighted unifrac distance (B). Similarity of the composition of bacterial flora between the samples increases as the distance between the dots decreases. Adult dogs: ●, geriatric dogs: day 0, ■; day 21, ▲; day 42, ►.

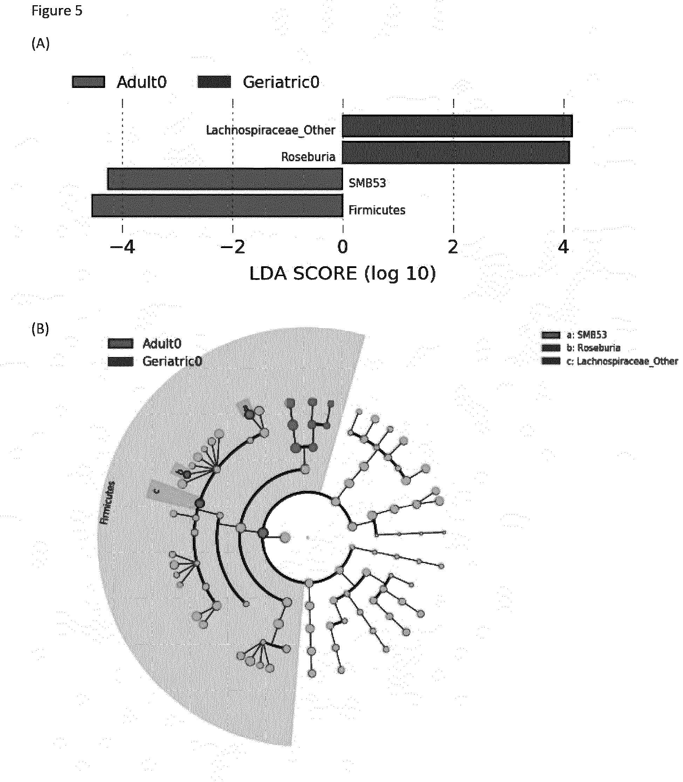

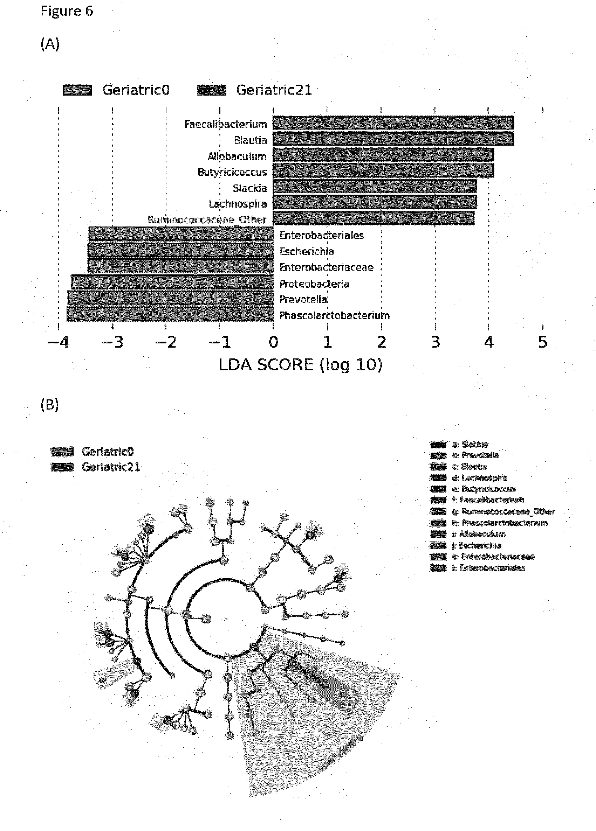

Next, the composition ratio of the intestinal bacterial flora was compared between the groups using the LEfSe method. Although an overall decrease was noted in the division Firmicutes, some Lachnospiraceae species were significantly higher in the geriatric group compared to the adult dogs (Figure5) Figure 5(A) shows Histograms of changes in the amount of bacterial species in each group. Figure 5(B) shows a cladogram of detected component bacterial specieis. The concentric circles represent Kingdom, Division, Class, Order, Family and Genus in the order from the center respectively, and one dot represents one bacterial species. Bacterial species that increased in the geriatric dogs and those that increased in the adult dogs are presented in different shades. Treatment effects of diet were additionally observed, while the dogs received the high fibre test diet significantly increased *Faecalibacterium, Blautia,* and *Lachnospira,* and significantly decreased Enterobacteriales, including *Escherichia* were detected in faeces (Figure6). Figure 6(A) shows histograms of changes in the amount of bacterial species in each group. Figure 6(B) shows a cladogram of detected component bacterial species. Bacterial species that characteristically increased on days 0 and 21 are presented in different shades. No bacterial species characteristically increased on day 42.

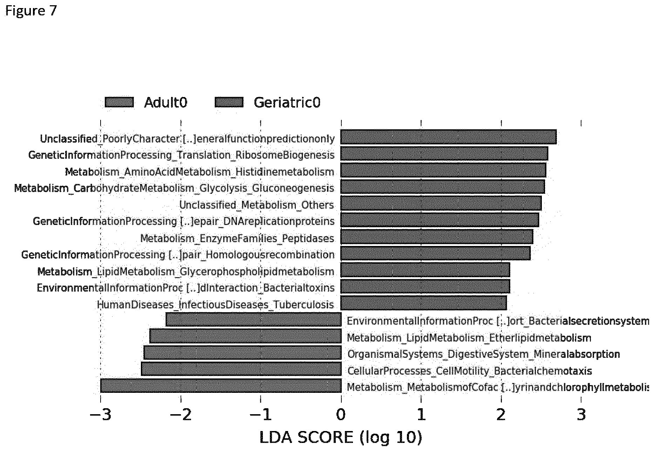

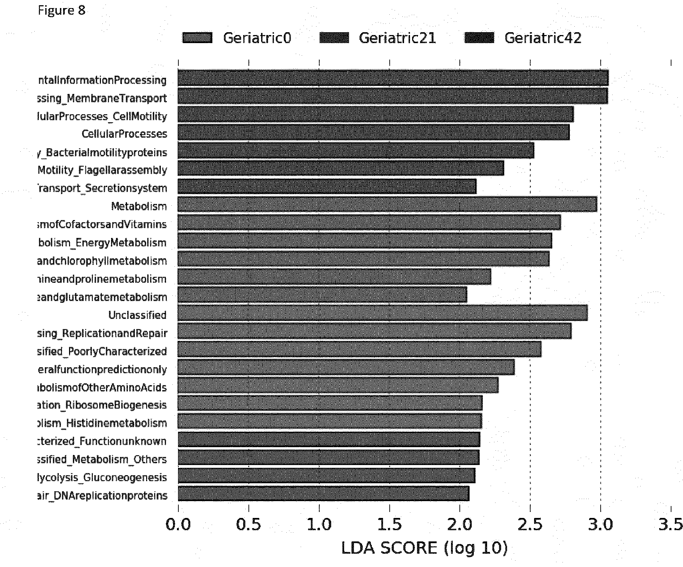

When the a level on the Kruskal-Wallis test employing LEfSe was set at 0.05 to compare the functional gene composition predicted by PICRUSTt at KEGG Orthology class 3 level, ether lipid metabolism-related genes decreased, and histidine metabolism-related enzymes, peptidase, and glycerophospholipid and glucose metabolism-related enzymes was higher in the geriatric group compared with those in the adult group, demonstrating detection of a difference in nutrient metabolism-related genes (Figure 7). The functions that increased in the geriatric and adult dogs are presented in different shades. Moreover, when dogs were fed the high fibre test diet for a period of 21 days an increase in genes related to the metabolism of several amino acids was detected, including proline, arginine, alanine, aspartic acid, and glutamic acid (Figure 8). The functions that characteristically increased on days 0, 21, and 42 are presented in different shades. Where day 0 represented faeces microbiome composition after 21 days on base diet, day 21 represented faeces microbiome composition 21 days after switching to the test formulation diet 'B', and day 42 represented faeces microbiome composition after a further 21 days following the return to base diet.

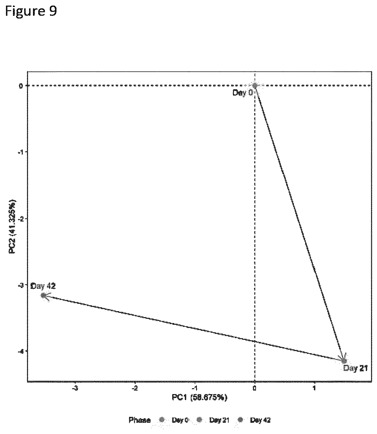

Multiple factor analysis (FactoMineR library) of the compositional ratios (relative abundance) of bacterial 'clusters' or taxa detected in the 5 dogs throughout the period of diet change resulted in a visual spider plot representative of the gross compositional features of the total microbiota observed within the cohort (Figure 9). The analysis suggested a clear movement by phase (diet) at the three study timepoints (at Day 0 (3 weeks after start of feeding base maintenance diet); Day 21 (3 weeks after the transition of dogs to the high fibre test diet 'B') and Day 42 (3 weeks after the return to feeding the base maintenance diet)).

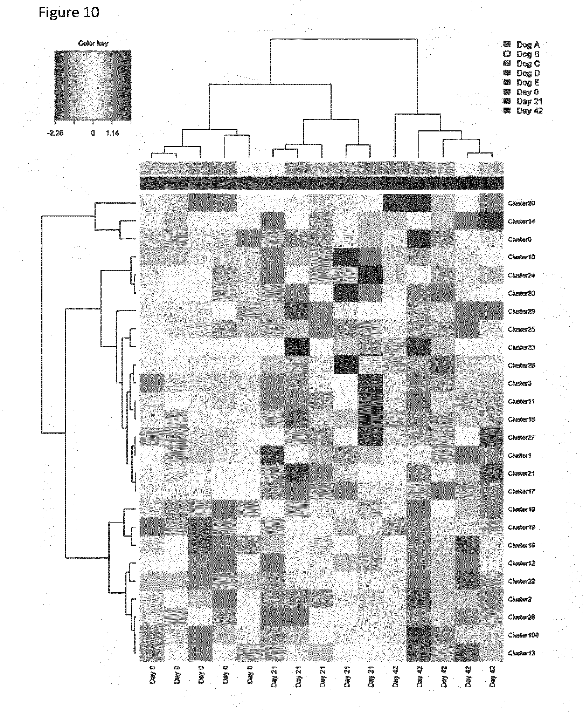

Comparisons of the relative abundance (composition) of the 105 clusters (bacterial taxa) observed within the cohort by partial least squares discriminant analysis (PLS-DA), enabled detection of a subset of bacterial clusters. These bacterial clusters or taxa represented those most influential in the correlation plot created by the hierarchical clustering algorithm (Data available on request). Bacterial clusters within this subset were defined by having a variable importance in projection (VIP) score of greater than 1. A total of 26 bacterial taxa (almost 25% of the abundant taxa detected) were observed to possess VIP scores equal to or greater than 1. This subset of 26 bacterial taxa represented those most influential in describing the interaction of the microbiota with diet. Figure 10 is a PLS-DA correlation plot indicating correlations in the relative composition of the faecal microbiota based on 26 bacterial clusters (taxa) in 3 day pooled faeces samples from the 5 dogs individual dogs after 3 weeks of feeding Diet A (Day 0), after transition to Diet B (Day 21) and after the subsequent return to base Diet A (Day 42). When the individual dog effect was removed, the diet effect, which was focused on the 26 taxa described in the PLS-DA (Table 5) was clearly observed in the clusters generated.

### Discussion

In this study detectable changes in the community composition of the intestinal bacterial flora (the microbiota), functional gene composition (the microbiome), and blood amino acid fractions were observed in dogs fed a high fibre test diet compared to when they received a premium commercially available base diet.

A subset of 26 bacterial taxa represented those most influential in describing the compositional changes in the microbiota with diet and a further three weeks after the return to baseline diet compositional changes were again consistently observed suggesting that treatment effect was indeed related to the diet change. These findings describe an influence of ingredients on the gut microbiome of dogs even between diets of similar format and macronutrient composition and suggest that may induce longer term influences on the microbiota in dogs may be possible through a nutritionally optimised diet plan.

The intestinal bacterial flora community composition ratio was significantly altered by feeding the high fibre test diet. *Faecalibacterium, Blautia, Lachnospira,* and Ruminococcaceae increased after feeding the high fibre test diet. These species all belong to Clostridium cluster IV&XIVa and function in the homeostasis of the digestive tract through short-chain fatty acid production (Schmitz 2016; Honneffer et al., 2014).

Short-chain fatty acids play an important role in induction of regulatory T cell differentiation in the intestinal mucosa; however, no major change was noted in the lymphocyte subsets, including peripheral blood CD4+ and Foxp3+ lymphocytes, i.e., regulatory T cells. This may have been due to the influence of significant increases in genes related to the metabolism of carbohydrates and fatty acids, such as butyric acid, although short-chain fatty acid-producing bacteria increased. On the other hand, Enterobacteriales species, including *Escherichia,* which are known to increase widely in the entire digestive tract with age (Honneffer et al., 2014), were decreased when the animals received the high fibre test diet, suggesting that the test diet in this study improved the intestinal environment at the bacterial flora level. Thus, it is expected to help prevent digestive disease and improve gastrointestinal resilience.

Changes in the functional gene composition induced by the high fibre test diet were investigated. Genes related to the metabolism of several amino acids, such as proline, arginine, alanine, aspartic acid, and glutamic acid, increased when dogs received the high fibre test diet. This increase in metabolism-related genes is generally considered beneficial for the host, but serum aspartic acid, alanine, arginine, and proline decreased after feeding the diet for elderly dogs.

The levels of several amino acid fractions, such as serine, glycine, and carnosine, were lower in the geriatric dogs than in the adult group. Treatment effects of diet were also observed in the circulating amino acid levels with reductions in aspartic acid, serine, sarcosine, proline, glycine, a amino butyric acid, methionine, phenylalanine, 1-&3-methylhistidine, carnosine, ornithine, and arginine observed when the animals received the the the high fibre test diet.

Dietary treatment effects were observed on the community composition ratios of the intestinal bacterial microbiota when the dogs received the high fibre test diet. Noteably, significantly increased levels of multiple microorganisms associated with health in other mammals were increased when the animals were receiving the high fibre test diet. These included *Faecalibacterium, Blautia, Lachnospira,* and Ruminococcaceae species all of which belong to the Clostridium cluster IV&XIVa. Increased levels of these bacteria are associated with short chain fatty acid production, acidification of the gastrointestinal lumen and with enhanced energy availability and metabolism of indigestible carbohydrates. Hence, they are indicators of enhanced gastrointestinal resilience to infection by pathogens and opportunistically pathogenic species such as those from the Proteobacteria and in particular the Enterobacteriaceae groups which includes many bacterial pathogens and opportunistically pathogenic organisms. As Gram negative species these are inherently acid sensitive and are frequently outcompeted in an environment with high SCFA levels. In the reported study these treatment effects indicative of gastrointestinal resilience were also detected with significantly decreased Enterobacteriales, including *Escherichia* observed in faeces when the animals were in receipt of the test diet. Detectable changes in the composition of the microbiome in terms of functional genes predicted from the microbiota using PICRUST and in serum amino acid fractions while the dogs received the high fibre test diet were also observed. While all of the dogs involved in the study were healthy and hence clinical effects of these changes were not addressed during this study, changes in intestinal bacterial microbiota and in the microbiome are associated with gastrointestinal health and for many chronic diseases in the medical and veterinary fields. A combination of 26 bacterial taxa was most descriptive of the influence of diet on the microbiota. Feeding of the high fibre test diet increased the relative abundance of fourteen bacterial sequence clusters, which, when designated to bacterial taxa through database searches included representatives from a bacterial health signature for including *Blautia* and *Turicibacter.* The high fibre test diet assessed during this study therefore represents a suitable dietary intervention for enhancement of the gastrointestinal microbiota and the gut microbiome

The main interactions among epithelium cells, mucus barrier, immune cells, and intestinal bacterial flora are schematically presented. A: State with maintenance of homeostasis. Diverse bacterial species are present in the lumen, and dendritic cells recognized them directly or through M cells to distinguish whether they are pathogenic, followed by immunological elimination (mucin and antimicrobial peptide production mediated by IL-22) or anti-inflammatory reactions (induction and differentiation of regulatory T cells). Short-chain fatty acids produced by intestinal bacterial flora, especially Clostridium cluster IV & XIVa, strengthen the mucosal barrier, such as by promotion of adhesion between intestinal epithelium cells (tight junction) and mucin production, and promote regulatory T cell differentiation. B: State with changes in intestinal bacterial flora inducing chronic enteritis. Opportunistic pathogens (yellow) and pathogenic bacteria (red) relatively increase and the diversity is lost. It is considered that in this state, dendritic cells promote differentiation to Th1 and Th17 cells via antigen presentation, and large amounts of inflammatory cytokines, such as IL-17, IL-22, and IFN γ, are produced, inducing enteritis. Moreover, bacterial species belonging to Clostridium cluster IV & XIVa decrease, thereby decreasing short-chain fatty acid production and the induction of regulatory T cell differentiation. The formation of the mucosal barrier subsequently weakens, and mucosal invasion of pathogens increases and aggravates enteritis, creating a vicious cycle (cited from ref. 3, partially modified).

**Table 5 Bacterial signature descriptive of the dietary transition**

| **Enriched at Day** | **Diet** | **Position in signature (****Fig.10****)** | **Cluster** | **Bacterial signature** |
|---|---|---|---|---|
| 42 | Return to control | 1 | Cluster 30 | p_Bacteroidetes; c_Bacteroidia; o_Bacteroidales; f_[Paraprevotellaceae]; genus novel |
| 42 | Return to control | 2 | Cluster 14 | p_Firmicutes; c_Erysipalatrichi; o_Erysipelotrichales; f_Erysipelotrichaceae; g_Holdemanella [Eubacterium]; s_biforme |
| 42 | Return to control | 3 | Cluster 0 | p_Firmicutes; c_Clostridia; o_Clostridiales; f_Costridiaceae; g_Clostridium; s_hiranonis |
| 21 | High fibre test diet | 4 | Cluster 10 | p_Firmicutes; c_Clostridia; o_Costridiales; f_Lachnospiraceae; g_Blautia; s_producta |
| 21 | High fibre test diet | 5 | Cluster 14 | p_Bacteroidetes; c_Bacteroidia; o_Bacteroidales; f_Bacteroidaceae; g_Bacteroides; s_plebeius |
| 21 | High fibre test diet | 6 | Cluster 20 | p_Firmicutes; c_Clostridia; q_Clostridiales; f_Lachncsplraceae; g_Blautla |
| 21 | High fibre test diet | 7 | Cluster 29 | p_Firmicutes; c_Clostridia; o_Costridiales; f_Clostridiaceae; g_Clostridium |
| 21 | High fibre test diet | a | Cluster 25 | p_Firmicutes; c_Clostridia; o_Clostridiales; f_Lachnospiraceae |
| 21 | High fibre test diet | 9 | Cluster 23 | p_Firmicutes; c_Clostridia; o_Costridiales; f_Lachnosplraceae |
| 21 | High fibre test diet | 10 | Cluster 26 | p_Firmicutes; c_Erysipelatrichi; o_Erysipelotrichales; f_Eryslpelotrichaceae; g_; s_ |
| 21 | High fibre test diet | 11 | Cluster 3 | p_Firmicutes; c_Erysipelotrichi; o_Erysipelotrichales, f_Erysipelotrichacae; g_Catenibacterium; s_ |
| 21 | High fibre test diet | 12 | Cluster 11 | p_Firmicutes; c_Erysipelotrichi; o_Eryslpelotrichales; f_Erysipelotrichaceae; g_Allobaculum: s_ |
| 21 | High fibre test diet | 13 | Cluster 15 | p_Firmicutes; c_Clostridia; o_Clostridiales; f_Lachnospiraceae; g_Blautia; s_producta |
| 21 | High fibre test diet | 14 | Cluster 27 | p_Fusobacteria; c_Fusobacteriia; o_Fusobacteriales; f_Fusobacteriaceae; g_Fusobacterium, s_ |
| 21 | High fibre test diet | 15 | Cluster 1 | p_Firmicutes; c_Bacilli; o_Turicibacterales; f_Turicibacteraceae; g_Turicibacter; s_ |
| 21 | High fibre test diet | 16 | Cluster 21 | p_Firmicutes; c_Clostridia; o_Clostridiales; f_Lachnosplraceae; g_Clostridium |
| 21 | High fibre test diet | 17 | Cluster 17 | p_Firmicutes; c_Bacilli; o_Turicibacterales; f_Turidbacteraceae; g_Turicibacter; s_ |
| 0 | Base control diet | 18 | Cluster 18 | p_Fusobacteria; c_Fusobacteriia; o_Fusobacteriales f_Fusobacteriaceae; g_Fusobacterium; s_ |
| 0 | Base control diet | 19 | Cluster 19 | p_Firmicutes; c_Clostridia; o_Costridiales; f_Veillonellaceae; g_Phascolarctobacterium; s_ |
| 0 | Base control diet | 20 | Cluster 16 | p_Firmicutes; c_Erysipelotrichi; o_Erysipelotrichales; f_Erysipelotrichaceae |
| 0 | Base control diet | 21 | Cluster 11 | p_Firmicutes; c_Clostridia; o_Costridiales; f_Lachnospiraceae |
| 0 | Base control diet | 22 | Cluster 22 | p_Actinobacteria; c_Coriobacteriia; o_Coriobacteriales; f_Coriobacteriaceae; g_Slackia; s_ |
| 0 | Base control diet | 23 | Cluster 2 | p_Firmicutes c_Clostridia; o_Costridiales; f_Velllonellaceae; g_Megamonas; s_ |
| 0 | Base control diet | 14 | Cluster 28 | p_Firmicutes; c_Clostridia; o_Clostridiales; f_Lachnospiraceae; g_; s_ |
| 0 | Base control diet | 25 | Cluster 100 | p_Firmicutes; c_Clostridia; o_Clostridiales; f_Ruminococcaceae; g_Oscillospira; s_ |
| 0 | Base control diet | 26 | Cluster 13 | p_Bacteroidetes; c_Bacteroidia; o_Bacteroidales; f_Bacteroidaceae; g_Bacteroides; s_ |

**Table 6. Characteristics of each group**

| | Adult | Geriatric | | | *P*-value* |
|---|---|---|---|---|---|
| | day0 | day0 | day21 | day42 | |
| Sex | | | | | |
| ♂ | 2 | 2 | - | - | 1.000 |
| ♀ | 3 | 3 | - | - | |
| Body weight (kg) | 11.1 ± 1.1 | 11.8 ± 1.9 | 11.6 ± 2.0 | 12.26 ± 2.0 | 0.638 |
| Age (months) | 36.2 ± 1.3 | 114.2 ± 20.7 | - | - | 0.009 |

| | | | | | |
|---|---|---|---|---|---|
| All values are presented as the mean ± standard deviation. *Kruskal-Wallis test | | | | | |

**Table 7. Blood test results of each group**

| | | Adult | Geriatric | | | *P*-value* |
|---|---|---|---|---|---|---|
| | | day0 | day0 | day21 | day42 | |
| | | | | | | |
| TP | g/dL | 6.0 ± 0.6 | 6.4 ± 0.2 | 6.2 ± 0.2 | 6.5 ± 0.3 | 0.141 |
| ALB | g/dL | 2.8 ± 0.2 | 2.9 ± 0.1 | 2.9 ± 0.1 | 2.9 ± 0.2 | 0.899 |
| T-BIL | mg/dL | 0.1 ± 0.0 | 0.1 ± 0.0** | 0.0 ± 0.0 | 0.1 ± 0.0 | 0.042 |
| AST | U/L | 33.4 ± 4.3 | 27.2 ± 11.8 | 24.6 ± 5.1 | 21.0 ± 4.3**^{,‡} | 0.038 |
| ALT | U/L | 35.6 ± 10.3 | 74.8 ± 54.0 | 51.4 ± 19.0 | 50.6 ± 17.5 | 0.188 |
| LDH | U/L | 38.6 ± 5.6 | 32.4 ± 8.6 | 111.4 ± 25.4**,^{†} | 100.0 ± 17.7** | 0.002 |
| ALP | U/L | 305.4 ± 184.5 | 315.6 ± 222.7 | 195.4 ± 128.6 | 352.4 ± 155.7 | 0.288 |
| GGT | U/L | 7.2 ± 2.5 | 8.4 ± 4.9 | 6.2 ± 2.7 | 5.6 ± 1.5 | 0.393 |
| CK | U/L | 119.8 ± 21.3 | 73.4 ± 19.9 | 129.0 ± 61.9^{†} | 85.2 ± 11.4 | 0.026 |
| AMY | U/L | 828.6 ± 168.5 | 950.2 ± 288.7 | 859.2 ± 319.8 | 997.4 ± 370.6 | 0.807 |
| v-LIP | U/L | 32.2 ± 15.3 | 48.8 ± 12.1 | 39.4 ± 11.7 | 55.6 ± 21.1 | 0.133 |
| BUN | mg/dL | 12.8 ± 3.2 | 11.6 ± 3.2 | 13.6 ± 2.2 | 14.4 ± 1.5 | 0.449 |
| CRE | mg/dL | 0.7 ± 0.1 | 0.6 ± 0.1 | 0.6 ± 0.1**^{,†} | 0.5 ± 0.1** | 0.046 |
| T-CHO | mg/dL | 172.8 ± 43.6 | 202.4 ± 7.2 | 185.0 ± 35.6 | 239.4 ± 64.9 | 0.123 |
| TG | mg/dL | 82.0 ± 40.7 | 173.0 ± 83.4" | 57.6 ± 24.3^{†} | 116.2 ± 54.4 | 0.028 |
| Na | mmol/L | 147.6 ± 2.3 | 146.4 ± 0.9 | 143.0 ± 1.2**^{,†} | 148.6 t 0.5$ | 0.003 |
| Cl | mmol/L | 117.4 ± 1.7 | 114.6 ± 1.5** | 119.4 ± 1.5^{†} | 114.0 ± 1.4**^{,‡} | 0.002 |
| K | mmol/L | 3.8 ± 0.1 | 3.9 ± 0.3 | 4.1 ± 0.2 | 4.2 ± 0.3 | 0.120 |
| Ca | mg/dL | 10.6 ± 0.5 | 10.6 ± 0.3 | 10.3 ± 0.3 | 10.4 ± 0.2 | 0.308 |
| PHS | mg/dL | 3.7 ± 0.6 | 3.7 ± 0.3 | 3.5 ± 0.4 | 4.0 ± 0.4 | 0.351 |
| GLU | mg/dL | 104.4 ± 19.3 | 97.4 ± 11.8 | 99.6 ± 4.3 | 80.4 ± 6.8**^{,‡} | 0.043 |
| TBA | umol/L | 6.3 ± 3.5 | 7.6 ± 4.6 | 4.0 ± 4.1 | 7.2 ± 6.6 | 0.618 |
| WBC | /uL | 10560 ± 3811 | 6000 ± 1453** | 6220 ± 2640** | 6120 ± 1950** | 0.082 |
| Gra | /uL | 6504 ± 2742 | 3402 ± 1333 | 3840 ± 2014 | 3863 ± 1736 | 0.147 |
| Mon | /uL | 922 ± 339 | 567 ± 91** | 597 ± 209** | 481 ± 147** | 0.021 |
| Lym | /uL | 1993 ± 454 | 1057 ± 166" | 916 ± 226** | 869 ± 154** | 0.006 |
| CD3⁺ | /uL | 1503 ± 345 | 639 ± 222** | 726 ± 222** | 637 ± 145** | 0.012 |
| CD4⁺ | /uL | 941 ± 191 | 337 ± 111** | 406 ± 120**^{,†} | 341 ± 84**^{,‡} | 0.010 |
| CD8⁺ | /uL | 562 ± 222 | 302 ± 121" | 320 ± 118** | 296 ± 90** | 0.136 |
| Foxp3⁺ | /uL | 43 ± 5 | 22 ± 9** | 20 ± 7** | 28 ± 14** | 0.022 |
| CD21⁺ | /uL | 238 ± 170 | 124 ± 91 | 111 ± 58 | 113 ± 63 | 0.555 |
| RBC | 10⁴/uL | 742 ± 65 | 778 ± 99 | 697 ± 70 | 681 ± 63 | 0.278 |
| HGB | g/dL | 17.5 ± 1.5 | 17.7 ± 1.8 | 15.8 ± 1.5 | 16.1 ± 1.4 | 0.186 |
| HCT | % | 50.8 ± 4.3 | 51.6 ± 5.4 | 46.3 ± 4.4 | 45.6 ± 4.1 | 0.080 |
| MCV | fL | 68.5 ± 1.9 | 66.5 ± 3.4 | 66.4 ± 2.5 | 67.0 ± 2.1 | 0.514 |
| MCH | pg | 23.6 ± 0.5 | 22.9 ± 1.4 | 22.7 ± 1.2 | 23.7 ± 1.2 | 0.678 |
| MCHC | g/dL | 34.4 ± 0.5 | 34.4 ± 0.5 | 36.2 ± 4.9 | 35.4 ± 0.7 | 0.127 |
| PLT | 10⁴/uL | 36.8 ± 6.2 | 34.2 ± 4.2 | 31.9 ± 6.9 | 36.2 ± 6.9 | 0.656 |

| | | | | | | |
|---|---|---|---|---|---|---|
| All values are presented as the mean ± standard deviation. *Kruskal-Wallis test; ** Significant difference vs. adult group (Dunnett's test); t Significant difference vs. day 0 (Friedman test); ‡Significant difference vs. day 21 (Friedman test) | | | | | | |

**Table 8. Serum amino acid fractions in each group**

| | Adult | Geriatric | | | P-value* |
|---|---|---|---|---|---|
| | day0 | day0 | day21 | day42 | |
| Taurine | 250.9 ± 36.1 | 225.6 ± 33.4 | 221.0 ± 32.6 | 195.2 ± 20.3** | 0.073 |
| Aspartic acid | 11.6 ± 2.6 | 9.9 ± 0.9 | 7.5 ± 2.3** | 6.0 ± 1.2** | 0.009 |
| Hydroxyproline | 35.8 ± 9.5 | 27.0 ± 8.7 | 24.9 ± 5.0 | 31.0 ± 11.8 | 0.315 |
| Threonine | 185.1 ± 29.9 | 207.1 ± 49.2 | 213.0 ± 62.7 | 228.9 ± 66.6 | 0.643 |
| Serine | 205.3 ± 30.9 | 153.7 ± 26.4** | 138.1 ± 13.2** | 177.4 ± 40.4^{‡} | 0.023 |
| Asparagine | 58.9 ± 7.7 | 52.3 ± 9.5 | 47.6 ± 5.2 | 51.6 ± 10.1 | 0.216 |
| Glutamic acid | 51.3 ± 6.0 | 53.6 ± 11.4 | 44.2 ± 11.6 | 41.8 ± 4.1 | 0.092 |
| Glutamine | 680.7 ± 44.6 | 712.8 ± 103.5 | 642.5 ± 88.6 | 567.9 ± 72.0 | 0.090 |
| Sarcosine | 8.4 ± 1.1 | 5.9 ± 5.9 | 1.1 ± 2.5** | 9.2 ± 4.4^{‡} | 0.033 |
| α-Aminoadipic acid | 0.5 ± 1.0 | 0.4 ± 0.9 | 0.0 ± 0.0 | 0.5 ± 1.2 | 0.768 |
| Proline | 293.6 ± 47.2 | 256.5 ± 57.2 | 158.0 ± 25.7** | 249.9 ± 80.6^{‡} | 0.031 |
| Glycine | 343.8 ± 33.3 | 238.9 ± 27.4** | 226.8 ± 21.8** | 229.6 ± 29.3** | 0.010 |
| Alanine | 623.6 ± 113.8 | 683.1 ± 119.6 | 508.5 ± 63.2 | 571.3 ± 90.5 | 0.089 |
| Citrulline | 56.8 ± 16.0 | 47.0 ± 11.8 | 39.8 ± 13.9 | 47.4 ± 16.9 | 0.432 |
| α-Aminobutyric acid | 25.4 ± 3.7 | 33.8 ± 6.2 | 23.0 ± 8.3^{†} | 34.6 ± 8.6^{‡} | 0.030 |
| Valine | 185.9 ± 23.1 | 214.2 ± 49.1 | 165.7 ± 16.9 | 227.1 ± 35.1^{‡} | 0.046 |
| Cystine | 6.3 ± 2.9 | 8.1 ± 2.8 | 11.9 ± 2.6**^{,†} | 3.3 ± 2.3^{‡} | 0.011 |
| Cystathionine | 5.9 ± 1.3 | 5.3 ± 1.3 | 4.7 ± 1.8 | 4.9 ± 1.1 | 0.418 |
| Methionine | 109.1 ± 23.4 | 98.8 ± 28.3 | 51.8 ± 4.4**^{,†} | 109.6 ± 37.2^{‡} | 0.016 |
| Isoleucine | 62.5 ± 10.7 | 78.0 ± 15.0 | 62.4 ± 9.5 | 74.8 ± 7.8 | 0.115 |
| Leucine | 124.2 ± 16.2 | 153.2 ± 35.6 | 134.1 ± 19.4 | 153.5 f 13.1 | 0.106 |
| Tyrosine | 63.2 ± 8.5 | 68.7 ± 16.8 | 44.5 ± 3.4 | 77.5 ± 25.6^{‡} | 0.036 |
| Phenylalanine | 60.8 ± 6.2 | 70.9 ± 6.6 | 60.1 ± 6.0^{†} | 69.9 ± 7.9** | 0.048 |
| y-Arnino β-hydroxy butyric acid | 0.0 ± 0.0 | 0.0 ± 0.0 | 0.0 ± 0.0 | 0.0 ± 0.0 | ND |
| β-Alanine | 0.6 ± 1.4 | 0.0 ± 0.0 | 0.0 ± 0.0 | 0.0 ± 0.0 | 0.392 |
| β-Amino-iso-butyric acid | 0.0 ± 0.0 | 0.0 ± 0.0 | 0.0 ± 0.0 | 0.0 ± 0.0 | ND |
| y-Aminobutyric acid | 0.0 ± 0.0 | 0.0 ± 0.0 | 0.0 ± 0.0 | 0.0 ± 0.0 | ND |
| Monoethanolamine | 12.6 ± 4.0 | 11.2 ± 1.2 | 9.0 ± 2.1 | 9.5 ± 0.8 | 0.157 |
| Homocystine | 0.0 ± 0.0 | 0.0 ± 0.0 | 0.0 ± 0.0 | 0.0 ± 0.0 | ND |
| Histidine | 85.7 ± 4.7 | 90.2 ± 13.1 | 79.2 ± 10.3 | 82.1 ± 7.1 | 0.637 |
| 3-Metylhistidine | 7.4 ± 1.4 | 4.2 ± 2.4 | 4.3 ± 2.5** | 3.9 ± 2.2^{‡} | 0.009 |
| 1-Metylhistidine | 9.7 ± 2.4 | 6.7 ± 1.6 | 3.1 ± 2.8** | 6.9 ± 1.0^{‡} | 0.006 |
| Carnosine | 30.7 ± 3.3 | 25.0 ± 3.7** | 24.7 ± 4.1** | 28.3 ± 3.7 | 0.075 |
| Anserine | 0.0 ± 0.0 | 0.0 ± 0.0 | 0.0 ± 0.0 | 0.0 ± 0.0 | ND |
| Tryptophan | 76.2 ± 7.2 | 93.2 ± 21.3 | 81.1 ± 23.3 | 101.3 ± 14.4 | 0.101 |
| Hydroxylysine | 0.0 ± 0.0 | 0.0 ± 0.0 | 0.0 ± 0.0 | 0.0 ± 0.0 | ND |
| Ornithine | 28.8 ± 5.4 | 25.1 ± 8.9 | 13.2 ± 1.9**^{,†} | 22.3 ± 6.5^{‡} | 0.010 |
| Lysine | 165.9 ± 23.1 | 214.4 ± 35.1** | 154.9 f 13.4 | 190.4 ± 43.3 | 0.055 |
| Arginine | 245.4 ± 44.9 | 214.9 ± 15.4 | 157.6 ± 18.6**^{,†} | 201.2 ± 39.9^{‡} | 0.014 |

| | | | | | |
|---|---|---|---|---|---|
| All values are presented as the mean ± standard deviation (nmol/mL). *Kruskal-Wallis test; ** Significant difference vs. adult group (Dunnett's test); t Significant difference vs. day 0 (Friedman test); ‡ Significant difference vs. day 21 (Friedman test) | | | | | |

**Table 9. a diversity index of intestinal bacterial flora in each group**

| | Adult | Geriatric | | | *P*-value* |
|---|---|---|---|---|---|
| | day0 | day0 | day21 | day42 | |
| PD | 4.40 ± 0.58 | 4.96 ± 0.32 | 4.82 ± 0.38 | 4.35 ± 0.62 | 0.240 |
| Chaol | 66.7 ± 9.9 | 74.3 ± 7.4 | 71.5 ± 5.6 | 63.5 ± 11.4 | 0.287 |
| out | 63.2 ± 10.8 | 71.3 ± 5.5 | 70.1 ± 5.5 | 61.1 ± 10.4 | 0.158 |
| Shannon | 3.77 ± 0.55 | 4.36 ± 0.29 | 4.28 ± 0.24 | 3.70 ± 0.38 | 0.057 |

| | | | | | |
|---|---|---|---|---|---|
| All values are presented as the mean ± standard deviation. *Kruskal-Wallis test | | | | | |

In addition, the invention further comprises the following numbered embodiments:
1. A composition suitable for administration to a companion animal, the composition comprising at least 3 ingredients selected from: green tea polyphenols of about 0.005 grams/day to about 0.165 grams/day, wheat of about 0.5 grams/day to about 33 grams/day, cellulose of about 0.2 grams/day to about 30.8 grams/day, chicory pulp and/or beet pulp to a total amount of about 0.1 grams/day to about 11.0 grams /day; tomato pomace (lycopene) of about 0.08 grams/day to about 2.2 grams/day, and fructooligosaccharides of about 0.025 grams/day to about 2.2 grams/day.
2. The composition of embodiment 1 comprising all of the ingredients.
3. The composition of embodiment 1 or 2 comprising at least one further ingredient selected from L-camitine, chondroitine sulfate, glucosamine, lutein and hydroxyproline collage.
4. The composition of embodiment 3 comprising all the further ingredients.
5. The composition of any one of the previous embodiments wherein the range of chicory pulp and/or beet pulp combined is present in the range of 0.1 to 6.6 grams/day.
6. The composition of any of the previous embodiments wherein the composition is a food that is nutritionally complete and at least a portion of the food comprises dry matter.
7. The composition of embodiment 7, wherein the nutrient profile of the food as a percentage of dry matter in the food comprises about 7.5% to about 19.8 % dietary fiber, about 10.0% to about 19.8% crude fat, about 2.5% to about 44.0% protein, and about 0.08% to about 4.4% indigestible protein.
8. The composition of any one of embodiments 1 to 5, wherein the composition is not nutritionally complete, and at least a portion of the food comprises dry matter.
9. The composition of embodiment 8, wherein the composition is a supplement, a topper or a booster.
10. The composition of either embodiments 8 or 9, wherein the nutrient profile of the food as a percentage of the dry matter in the composition is about 0.9% to about 99.9% dietary fiber, about 1.0% to about 19.8% crude fat, about 2.5% to about 44.0% protein, and about 0.08% to about 4.4% indigestible protein.
11. The composition of any one of the previous embodiments, wherein the companion animal is a canid.
12. The composition of the any one of the previous embodiments for use in a medicament.
13. The composition of any one of the previous embodiments for use in treating gastro-intestinal dysbiosis, soft stools, loose faeces or gastro-intestinal distress in a companion animal.
14. The composition of any one of the previous embodiments for use in altering the microbiome in a companion animal.
15. The composition of any one of the previous embodiments for use in altering or increasing CD3+ and/or CD4+ lymphocyte counts in a companion animal.
16. The composition of any one of the previous embodiments for use in altering or decreasing circulatory triglyceride levels in a companion animal.
17. A method of changing the microbiome of a companion animal by administering the composition of any of embodiments 1 to 12 to a companion animal.
18. The method of embodiment 17, wherein the microbiome is the gastro-intestinal microbiome of the companion animal.
19. The method of any one of embodiments 17 or 18, wherein the companion animal is a canid.
20. The method of any one of embodiments 17 to 19, wherein the companion animal of embodiments 17 and 18, or the canid of embodiment 19 is a senior or geriatric animal.
21. The method of embodiment 17, further comprising a first step of determining the health of the companion animal's microbiome, and the composition is administered to the companion animal when there is determination of a healthy or an unhealthy microbiome detected in the first step, preferably an unhealthy microbiome.
22. The method of embodiment 21, wherein the first step comprises detecting at least two, preferably at least three or at least four bacterial taxa in a sample obtained from the companion animal; wherein the presence of at least two, preferably at least three or at least four bacterial taxa is indicative of a healthy microbiome.
23. The method of embodiment 22, wherein the companion animal is a canid.
24. The method of embodiment 23, wherein the bacterial taxa are selected from at least two of the group Faecalibacterium, Blautia, Allobaculum, Butyricicoccus, Slackia, Lachnospira, Roseburia and Ruminococcaceae.
25. The method of embodiment 23, wherein the bacterial taxa are bacterial families selected from at least three of the group Lachnospiraceae, Bacteroidaceae, Clostridiaceae, Erysipelotrichaceae, and Turicibacteraceae.
26. The method of embodiment 23, wherein the bacterial taxa are bacterial genera selected from at least two of the group Blautia, Bacteroides, Clostridium, Catenibacterium, Allobaculum, Fusobacterium and Turicibacter.
27. The method of embodiment 22, wherein the first step comprises detecting at least two, preferably at least three or at least four bacterial taxa in a sample obtained from the companion animal; wherein the presence of at least two, preferably at least three or at least four bacterial taxa is indicative of an unhealthy microbiome.
28. The method of embodiment 27 wherein the companion animal is a canid.
29. The method of embodiment 28, wherein the bacterial taxa are selected from at least two of the group: Enterobacteriales, Escherichia, Enterobacteriaceae, Proteobacteria, Prevotella and Phascolartobacterium.
30. The method of embodiment 28, wherein the bacterial taxa are from the families selected from at least three of the group Fusobacteriaceae, Veillonellaceae, Erysipelotrichaceae, Lachnospiraceae, Coriobacteriaeae, Ruminococcaceae, Bacteriodaceae.
31. The method of embodiment 28, wherein the bacterial taxa are from bacterial genera selected from at least two of the group: Fusobacterium, Phascolarctobacterium, Slackia, Megoamonas, Oscillospira, Bacteroides.
32. The method of embodiment 21, wherein the first step comprises quantitating at least two, preferably at least three or at least four bacterial taxa in a sample obtained from the companion animal to determine their abundance; and comparing the determined abundance to the abundance of the same taxa in a control data set; wherein an increase or decrease in the abundance of the at least two, preferably at least three or at least four bacterial taxa relative to the control data set is indicative of an unhealthy microbiome.
33. The method of embodiment 32, wherein a decrease in determined abundance relative to the control data set is indicative of an unhealthy microbiome.
34. The method of embodiment 33, wherein the bacterial taxa are selected from at least two of the group Faecalibacterium, Blautia, Allobaculum, Butyricicoccus, Slackia, Lachnospira, Roseburia and Ruminococcaceae.
35. The method of embodiment 33, wherein the bacterial taxa are bacterial families selected from at least three of the group Lachnospiraceae, Bacteroidaceae, Clostridiaceae, Erysipelotrichaceae, and Turicibacteraceae.
36. The method of embodiment 33, wherein the bacterial taxa are bacterial genera selected from at least two of the group Blautia, Bacteroides, Clostridium, Catenibacterium, Allobaculum, Fusobacterium, Turicibacter.
37. The method of embodiment 32, wherein an increase in determined abundance relative to the control data set is indicative of an unhealthy microbiome.
38. The method of embodiment 37, wherein the bacterial taxa are selected from at least two of the group Enterobacteriales, Escherichia, Enterobacteriaceae, Proteobacteria, Prevotella and Phascolartobacterium.
39. The method of embodiment 37, wherein the bacterial taxa are families selected from at least three of the group Fusobacteriaceae, Veillonellaceae, Erysipelotrichaceae, Lachnospiraceae, Coriobacteriaeae, Ruminococcaceae, Bacteriodaceae.
40. The method of embodiment 37, wherein the bacterial taxa are from bacterial genera selected from at least two of the group: Fusobacterium, Phascolarctobacterium, Slackia, Megoamonas, Oscillospira, Bacteroides.
41. The method of any one of embodiments 32 to 40, wherein the companion animal is a canid.
42. The method of any one of embodiments 32 to 41, wherein the control data set comprises a data set from a companion animal, preferably a canid, at the same life stage.
43. The method of any one of embodiments 17 to 42, wherein the companion animal of any one of embodiments 17 to 18, 20, 21 to 22, 27, 32 to 40, 42 or the canid of any one of embodiments 19, 20, 23 to 26, 28 to 31, 41, 42, is a puppy.
44. The method of any one of embodiments 17 to 42, wherein the companion animal of any one of embodiments 17 to 18, 20, 21 to 22, 27, 32 to 40, 42, or the canid of any one of embodiments 19, 20, 23 to 26, 28 to 31, 41, 42, is an adult, senior or geriatric canid.
45. The method of any one of embodiments 17 to 44, further comprising a step of changing the microbiome composition of the companion animal of any one of embodiments 17 to 18, 20, 21 to 22, 27, 32 to 40, 42, 43 or the canid of any one of embodiments 19, 20, 23 to 26, 28 to 31, 41, 42, 43, 44.
46. The method of any one of embodiments 21 to 45, wherein the first step comprises determining the health of the microbiome of the companion animal of any one of embodiments 17 to 18, 20, 21 to 22, 27, 32 to 40, 42, 43, 45 or health of the microbiome of the of any one of embodiments 19, 20, 23 to 26, 28 to 31, 41, 42, 43, 44, 45, at at least two time points.
47. The method of embodiment 46, wherein the two time points are at least about 3 months apart.
48. The method of embodiment 47, wherein the two time points are at least about 6 months apart.
49. The method of any one of embodiments 22 to 48, wherein the sample is from the gastrointestinal tract.
50. The method of embodiment 49, wherein the sample is selected from a faecal sample, an ileal sample, a jejunal sample, a duodenal sample and a colonic sample.
51. The method of any one of embodiments 17 to 50, wherein the health of the microbiome is determined before and after administration of the composition.
52. The method of any one of embodiments 17 to 51, wherein the bacterial taxa of any one of embodiments 24, 27 to 29, and 32 to 34, or the bacterial families of any one of embodiments 25, 30, 35 and 39, or the bacterial genera of any one of embodiments and 26, 31, 36 and 40 is detected by means of DNA sequencing, RNA sequencing, protein sequence homology or a biological marker indicative of the bacterial taxa, clade, subtype, family or genera.
53. The method of any one of embodiments 19, 20, 23 to 26, 28, 29 to 31 and 41 to 45 wherein the canid is a dog.
54. The composition of any one of embodiments 1 to 16 for use to increase the numbers of *Faecalibacterium, Blautia,Allobaculum, Butyricicoccus, Slackia Lachnospira,* and Ruminococcaceae present in the gastro-intestinal tract or faeces of a companion animal compared to the number of said bacteria present in the companion animal before administration of the composition.
55. The composition of any one of embodiments 1 to 16 for use in reducing the numbers of *Fusobacterium,* in particular *Fusobacterium mortiferum* or a species from the family Mogibacteriaceae or *Escherichia*/*Shigella* or Mediterraneibacter, or *Clostridium perfringens* or *Clostridium difficile.*
56. The composition of any one of embodiments 1 to 16 for use in reducing the numbers of Enterobacteriales, Escherichia, Enterobacteriaceae, Proteobacteria, Preveotella and Phascolarctobacterium in the gastro-intestinal tract or faeces of a companion animal compared to the number of said bacteria present in the companion animal before administration of the composition.
57. A method for increasing the bacterial counts of at least one of the bacterial taxa in a selected from *Faecalibacterium, Blautia, Lachnospira,* and Ruminococcaceae, in the gastro-intestine and/or faeces of a companion animal by administering the composition of embodiment 1 and/or for reducing at least one of the bacteria in a group consisting of the numbers of *Fusobacterium,* in particular *Fusobacterium mortiferum* or a species from the family Mogibacteriaceae or *Escherichia*/*Shigella* or Mediterraneibacter or *Clostridium perfringens* or *Clostridium difficile.*
58. The composition of embodiment 1 for use to decrease the number of Enterobacteriales bacteria, preferably *Escherichia or Fusobacterium*, in particular *Fusobacterium mortiferum* or a species from the family Mogibacteriaceae or *Clostridium perfringens* or *Clostridium difficile.*
59. The composition of any one of embodiments 1 to 16 for use in increasing the gene expression of at least one of proline-, arginine-, alanine-, aspartic acid- and glutamic acid- related genes in a microbiome of a companion animal.
60. A method for increasing the gene expression of at least one of proline-, arginine-, alanine, aspartic acid- and glutamic acid- related genes in a microbiome of a companion animal by administering the composition of any one of embodiments 1 to 16 to said companion animal.
61. The composition of any one of embodiments 1 to 16 for use to change the circulating amino acid levels in a companion animal.
62. The composition of embodiment 61, wherein the circulating amino acid levels selected from the levels of aspartic acid, serine, sarcosine, proline, glycine, a amino butyric acid, methionine, phenylalanine, 1-& 3- methylhistidine, carnosine, ornithine, and arginine are reduced.
63. A method for changing the circulating amino acid levels in a companion animal, said method comprising reducing any one of the amino acids in a group comprising aspartic acid, serine, sarcosine, proline, glycine, a amino butyric acid, methionine, phenylalanine, 1-& 3- methylhistidine, carnosine, ornithine, and arginine in a companion animal by administering the composition of any one of embodiments 1 to 16.
64. A method of increasing or altering the CD3+ and/or the CD4+ lymphocyte counts in a companion animal by administering the composition of any one of embodiments 1 to 16 to the companion animal.
65. A method of decreasing or altering the circulatory triglyceride count in a companion animal by administering the composition of any one of embodiments 1 to 16 to the companion animal.

## Claims

1. A composition suitable for administration to a companion animal, the composition comprising at least 3 ingredients selected from: wheat of about 0.5 grams/day to about 33 grams/day, cellulose of about 0.2 grams/day to about 30.8 grams/day, chicory pulp and/or beet pulp to a total amount of about 0.1 grams/day to about 11.0 grams /day; tomato pomace (lycopene) of about 0.08 grams/day to about 2.2 grams/day, and fructooligosaccharides of about 0.025 grams/day to about 2.2 grams/day.

2. The composition of claim 1 comprising all of the ingredients.

3. The composition of claim 1 or 2 comprising at least one further ingredient selected from L-camitine, chondroitine sulfate, glucosamine, lutein and hydroxyproline collage, optionally comprising all the further ingredients.

4. The composition of any one of the previous claims wherein the range of chicory pulp and/or beet pulp combined is present in the range of 0.1 to 6.6 grams/day.

5. The composition of any of the previous claims wherein the composition is a food that is nutritionally complete and at least a portion of the food comprises dry matter.

6. The composition of claim 5, wherein the nutrient profile of the food as a percentage of dry matter in the food comprises about 7.5% to about 19.8 % dietary fiber, about 10.0% to about 19.8% crude fat, about 2.5% to about 44.0% protein, and about 0.08% to about 4.4% indigestible protein.

7. The composition of any one of claims 1 to 4, wherein the composition is not nutritionally complete, and at least a portion of the food comprises dry matter, optionally wherein the composition is a supplement, a topper or a booster.

8. The composition of any one of the previous claims, wherein the companion animal is a canid.

9. The composition of the any one of the previous claims for use in a medicament.

10. The composition of any one of the previous claims for use:
(i) in treating gastro-intestinal dysbiosis, soft stools, loose faeces or gastro-intestinal distress in a companion animal;
(ii) in a method of altering the microbiome in a companion animal;
(iii) in altering or increasing CD3+ and/or CD4+ lymphocyte counts in a companion animal; or
(iv) in altering or decreasing circulatory triglyceride levels in a companion animal.

11. The composition of any one of claims 1 to 10 for use:
(i) to increase the numbers of *Faecalibacterium*, *Blautia*, *Allobaculum*, *Butyricicoccus*, *Slackia*, *Lachnospira*, and *Ruminococcaceae* present in the gastro-intestinal tract or faeces of a companion animal compared to the number of said bacteria present in the companion animal before administration of the composition;
(ii) in reducing the numbers of *Fusobacterium*, in particular *Fusobacterium mortiferum* or a species from the family *Mogibacteriaceae* or *Escherichia*/*Shigella* or *Mediterraneibacter*, or *Clostridium perfringens* or *Clostridium difficile;*
(iii) in reducing the numbers of Enterobacteriales, Escherichia, Enterobacteriaceae, Proteobacteria, Preveotella and Phascolarctobacterium in the gastro-intestinal tract or faeces of a companion animal compared to the number of said bacteria present in the companion animal before administration of the composition; and/or
(iv) to decrease the number of Enterobacteriales bacteria, preferably *Escherichia* or *Fusobacterium*, in particular *Fusobacterium mortiferum* or a species from the family Mogibacteriaceae or *Clostridium perfringens* or *Clostridium difficile.*

12. The composition of any one of claims 1 to 10 for use in increasing the gene expression of at least one of proline-, arginine-, alanine-, aspartic acid- and glutamic acid- related genes in a microbiome of a companion animal.

13. The composition of any one of claims 1 to 10 for use to change the circulating amino acid levels in a companion anima, optionally wherein the circulating amino acid levels selected from the levels of aspartic acid, serine, sarcosine, proline, glycine, α amino butyric acid, methionine, phenylalanine, 1- & 3-methylhistidine, carnosine, ornithine, and arginine are reduced.

14. Use of the composition of any one of claims 1-8 for:
(i) altering the microbiome in a companion animal;
(ii) altering or increasing CD3+ and/or CD4+ lymphocyte counts in a companion animal; or
(iii) altering or decreasing circulatory triglyceride levels in a companion animal.

15. Use of the composition of any one of claims 1-8 for:
(i) increasing the numbers of *Faecalibacterium*, *Blautia*, *Allobaculum*, *Butyricicoccus*, *Slackia*, *Lachnospira*, and *Ruminococcaceae* present in the gastro-intestinal tract or faeces of a companion animal compared to the number of said bacteria present in the companion animal before administration of the composition;
(ii) reducing the numbers of *Fusobacterium*, in particular *Fusobacterium mortiferum* or a species from the family *Mogibacteriaceae* or *Escherichia*/*Shigella* or *Mediterraneibacter*, or *Clostridium perfringens* or *Clostridium difficile;*
(iii) reducing the numbers of Enterobacteriales, Escherichia, Enterobacteriaceae, Proteobacteria, Preveotella and Phascolarctobacterium in the gastro-intestinal tract or faeces of a companion animal compared to the number of said bacteria present in the companion animal before administration of the composition; and/or
(iv) decreasing the number of Enterobacteriales bacteria, preferably *Escherichia* or *Fusobacterium*, in particular *Fusobacterium mortiferum* or a species from the family Mogibacteriaceae or *Clostridium perfringens* or *Clostridium difficile.*
